# EUROPEAN PATENT SPECIFICATION

(11) **EP 2 236 624 B1**
(45) Date of publication and mention of the grant of the patent: **07.11.2012**
(21) Application number: 07870602.5
(22) Date of filing: 09.08.2007
(51) Int. Cl.: C12Q 1/68, C12N 15/11

(54) **METHOD FOR IDENTIFYING THE GENOTYPE AND SUBTYPE OF HEPATITIS C VIRUS ON A BIOLOGICAL MICROCHIP**
VERFAHREN ZUR IDENTIFIZIERUNG DES GENOTYPS UND SUBTYPS VON HEPATITIS-C-VIRUS AUF EINEM BIOLOGISCHEN MIKROCHIP
PROCÉDÉ D'IDENTIFICATION DU GÉNOTYPE ET DU SOUS-TYPE DU VIRUS DE L'HÉPATITE C SUR UNE BIOPUCE

(43) Date of publication of application: 06.10.2010
(73) Proprietor: Federalnoe Gosudarstvennoe Byudzhetnoe Uchrezhdenie Nauki Institut Molekulyarnoi Biologi Im. V.A. Engelgardta Rossiiskoi Akademii Nauk, Moscaw 119991 (RU); UNIVERSITE PAUL SABATIER (TOULOUSE III), 31062 Toulouse Cedex (FR); CENTRE HOSPITALIER UNIVERSITAIRE DE TOULOUSE, 31059 Toulouse Cedex 9 (FR)
(72) Inventor: GRYADUNOV, Dmitry Alexandrovich, Moscow 117218 (RU); MIKHAILOVICH, Vladimir Mikhailovich, Moscow 127081 (RU); NICOT, Florence, F-31500 Toulouse (FR); DUBOIS, Martine, F-31270 Frouzins (FR); ZASEDATELEV, Alexandr Sergeevich, Moscow 117418 (RU); IZOPET, Jacques, F-31000 Toulouse (FR)
(74) Representative: Gallochat, Alain
(86) International application number: PCT/RU2007/000438
(87) International publication number: WO 2009/022939

(56) References cited:
- WO-A1-99/51781
- WO-A2-94/25601
- WO-A2-96/13590
- US-A1- 2004 191 768
- US-A1- 2004 191 768
- US-A1- 2007 020 665
- US-A1- 2007 020 665
- SANDRES-SAUNE K ET AL: "Determining hepatitis C genotype by analyzing the sequence of the NS5b region", JOURNAL OF VIROLOGICAL METHODS, ELSEVIER BV, NL, vol. 109, no. 2, 1 May 2003 (2003-05-01), pages 187-193, XP002425272, ISSN: 0166-0934, DOI: DOI:10.1016/S0166-0934(03)00070-3
- LAPERCHE S ET AL: "Comparison of hepatitis C virus NS5b and 5' noncoding gene sequencing methods in a multicenter study", JOURNAL OF CLINICAL MICROBIOLOGY, AMERICAN SOCIETY FOR MICROBIOLOGY, WASHINGTON, DC, US, vol. 43, no. 2, 1 January 2005 (2005-01-01), pages 733-739, XP002429959, ISSN: 0095-1137, DOI: DOI:10.1128/JCM.43.2.733-739.2005
- LAU J Y ET AL: "Distribution of hepatitis C virus genotypes determined by line probe assay in patients with chronic hepatitis C seen at tertiary referral centers in the United States. Hepatitis Interventional Therapy Group.", ANNALS OF INTERNAL MEDICINE 15 MAY 1996 LNKD- PUBMED:8610915, vol. 124, no. 10, 15 May 1996 (1996-05-15) , pages 868-876, XP009146658, ISSN: 0003-4819
- SYRIA LAPERCHE ET AL.: 'Comparison of Hepatitis C Virus NS5b and 5'Noncoding Gene Sequencing Methods in a Multicenter Study' JOURNAL OF CLINICAL MICROBIOLOGY vol. 43, no. 2, February 2005, pages 733 - 739, XP002429959

### SEQUENCE LISTING

<110> UCHREZHDENIE ROSSIISKOI AKADEHIJ NAUK INSTITUT NOI.EKULYARNOI BIOLOGIE IF. V.A. ENGELGARDTA RAN (IMB RAN) <120> METHOD OF IDENTIFICATION OF GENOTYPE AND SUBTYPE OF HEPATITIS C VIRUS ON BIOLOGICAL MICROCHIP
<160> 123
<170> PatentIn version 3.1
<210> 1
   <211> 14
   <212> DNA
   <213> Artificial Sequence
<220>
   <223> Genotype-specific oligonucleotide G1-1 immobilized on the biochip
<400> 1
   gcctgtcgag cygc 14
<210> 2
   <211> 15
   <212> DNA
   <213> Artificial Sequence
<220>
   <223> Genotype-specific oligonucleotide G1-2 immobilized on the biochip
<400> 2
   gcctgtcgag cygcr 15
<210> 3
   <211> 15
   <212> DNA
   <213> Artificial sequence
<220>
   <223> Genotype-specific oligonucleotide G1-3 immobilized on the biochip
<400> 3
   gcctgtmgag cygcr 15
<210> 4
   <211> 16
   <212> DNA
   <213> Artificial Sequence
<220>
   <223> Genotype-specific oligonucleotide G1-4 immobilized on the biochip
<400> 4
   ggctttayrt cggggg 16
<210> 5
   <211> 15
   <212> DNA
   <213> Artificial sequence
<220>
   <223> Genotype-specific oligonucleotide G2-1 immobilized on the biochip
<400> 5
   tacaggcgyt gycgc 15
<210> 6
   <211> 18
   <212> DNA
   <213> Artificial Sequence
<220>
   <223> Genotype-specific oligonucleotide G2-2 immobilized on the biochip
<220>
   <221> misc_feature
   <222> (7)..(7)
   <223> n is any of a, g, c, and t
<400> 6
   ctgcggntac aggcgttg 18
<210> 7
   <211> 18
   <212> DNA
   <213> Artificial Sequence
<220>
   <223> Genotype-specific oligonucleotide G2-3 immobilized on the biochip
<220>
   <221> misc_feature
   <222> (7)..(7)
   <223> n is any of a, g, c, and t
<400> 7
   ctgcggntac aggcgytg 18
<210> 8
   <211> 16
   <212> DNA
   <213> Artificial Sequence
<220>
   <223> Genotype-specific oligonucleotide G3-1 immobilized on the biochip
<400> 8
   ycttgtctgc ggagay 16
<210> 9
   <211> 16
   <212> DNA
   <213> Artificial Sequence
<220>
   <223> Genotype-specific oligonucleotide G3-2 immobilized on the biochip
<400> 9
   ggctttactg cggggg 16
<210> 10
   <211> 21
   <212> DNA
   <213> Artificial sequence
<220>
   <223> Genotype-specific oligonucleotide G4-1 immobilized on the biochip
<400> 10
   cgaggargag gtctaycagt g 21
<210> 11
   <211> 21
   <212> DNA
   <213> Artificial Sequence
<220>
   <223> Genotype-specific oligonucleotide G4-2 immobilized on the biochip
<400> 11
   cgaggargag gtmtaycagt g 21
<210> 12
   <211> 16
   <212> DNA
   <213> Artificial sequence
<220>
   <223> Genotype-specific oligonucleotide G4-3 immobilized on the biochip
<400> 12
   gtgacctrga gcccga 16
<210> 13
   <211> 16
   <212> DNA
   <213> Artificial sequence
<220>
   <223> Genotype-specific oligonucleotide G5-1 immobilized on the biochip
<400> 13
   gtgacttrca gcccga 16
<210> 14
   <211> 16
   <212> DNA
   <213> Artificial Sequence
<220>
   <223> Genotype-specific oligonucleotide G5-2 immobilized on the biochip
<400> 14
   gcacgctcct ggtgtg 16
<210> 15
   <211> 17
   <212> DNA
   <213> Artificial sequence
<220>
   <223> Genotype-specific oligonucleotide G-6 immobilized on the biochip
<400> 15
   tgacatgttg gtctgcg 17
<210> 16
   <211> 17
   <212> DNA
   <213> Artificial Sequence
<220>
   <223> Subtype-specific oligonucleotide 1a11 immobilized on the biochip
<400> 16
   cactgagagc gacatcc 17
<210> 17
   <211> 18
   <212> DNA
   <213> Artificial Sequence
<220>
   <223> Subtype-specific oligonucleotide 1ce1 immobilized on the biochip
<400> 17
   cactgaggct gatatccg 18
<210> 18
   <211> 17
   <212> DNA
   <213> Artificial Sequence
<220>
   <223> subtype-specific oligonucleotide 1a12 immobilized on the biochip
<400> 18
   yatccgtacg gaggagg 17
<210> 19
   <211> 18
   <212> DNA
   <213> Artificial Sequence
<220>
   <223> Subtype-specific oligonucleotide 1bd12 immobilized on the biochip
<400> 19
   yatccgtgtt gaggagtc 18
<210> 20
   <211> 18
   <212> DNA
   <213> Artificial Sequence
<220>
   <223> subtype-specific oligonucleotide 1a2 immobilized on the biochip
<400> 20
   catcaagtcc ctcacyga 18
<210> 21
   <211> 18
   <212> DNA
   <213> Artificial sequence
<220>
   <223> Subtype-specific oligonucleotide 1b2 immobilized on the biochip
<400> 21
   ataargtcgc tcacagag 18
<210> 22
   <211> 19
   <212> DNA
   <213> Artificial Sequence
<220>
   <223> subtype-specific oligonucleotide 1c2 immobilized on the biochip
<400> 22
   ccataaggtc tctcacaga 19
<210> 23
   <211> 16
   <212> DNA
   <213> Artificial Sequence
<220>
   <223> Subtype-specific oligonucleotide 1d2 immobilized on the biochip
<400> 23
   ataaagtcgc tcaccg 16
<210> 24
   <211> 20
   <212> DNA
   <213> Artificial Sequence
<220>
   <223> subtype-specific oligonucleotide 1e2 immobilized on the biochip
<400> 24
   atcaagtcyt tgactgaaag 20
<210> 25
   <211> 13
   <212> DNA
   <213> Artificial Sequence
<220>
   <223> subtype-specific oligonucleotide 1a31 immobilized on the biochip
<400> 25
   aggcccgrgc agc 13
<210> 26
   <211> 13
   <212> DNA
   <213> Artificial Sequence
<220>
   <223> Subtype-specific oligonucleotide 1a32 immobilized on the biochip
<400> 26
   aggcccargc agc 13
<210> 27
   <211> 15
   <212> DNA
   <213> Artificial sequence
<220>
   <223> subtype-specific oligonucleoticle 1b3 immobilized on the biochip
<400> 27
   gccwctgcrg cctgt 15
<210> 28
   <211> 14
   <212> DNA
   <213> Artificial Sequence
<220>
   <223> subtype-specific oligonucleotide 1bd3 immobilized on the biochip
<400> 28
   ccwctgcggc ctgt 14
<210> 29
   <211> 15
   <212> DNA
   <213> Artificial Sequence
<220>
   <223> subtype-specific oligonucleotide 1c3 immobilized on the biochip
<400> 29
   gccagtgcag cctgt 15
<210> 30
   <211> 15
   <212> DNA
   <213> Artificial Sequence
<220>
   <223> subtype-specific oligonucleotide 1e3 immobilized on the biochip
<400> 30
   caaggcccta gcagc 15
<210> 31
   <211> 14
   <212> DNA
   <213> Artificial sequence
<220>
   <223> Subtype-specific oligonucleotide 1d3 immobilized on the biochip
<400> 31
   gccatrgcrg cctg 14
<210> 32
   <211> 15
   <212> DNA
   <213> Artificial Sequence
<220>
   <223> Subtype-specific oligonucleotide 1a4 immobilized on the biochip
<400> 32
   ctaycgcagg tgccg 15
<210> 33
   <211> 14
   <212> DNA
   <213> Artificial Sequence
<220>
   <223> subtype-specific oligonucleotide 1b4 immobilized on the biochip
<400> 33
   gttatcgccg gtgc 14
<210> 34
   <211> 14
   <212> DNA
   <213> Artificial sequence
<220>
   <223> Subtype-specific oligonucleotide 1c4 immobilized on the biochip
<400> 34
   gctatcggcg atgc 14
<210> 35
   <211> 14
   <212> DNA
   <213> Artificial sequence
<220>
   <223> Subtype-specific oligonucleotide 1d4 immobilized on the biochip
<400> 35
   gctaccgtcg gtgc 14
<210> 36
   <211> 15
   <212> DNA
   <213> Artificial sequence
<220>
   <223> subtype-specific oligonucleotide 1e4 immobilized on the biochip
<400> 36
   tatcgcagat gccgt 15
<210> 37
   <211> 19
   <212> DNA
   <213> Artificial sequence
<220>
   <223> subtype-specific oligonucleotide 2ad1 immobilized on the biochip
<400> 37
   cagaahtgag gagtccata 19
<210> 38
   <211> 17
   <212> DNA
   <213> Artificial Sequence
<220>
   <223> subtype-specific oligonucleotide 2b11 immobilized on the biochip
<400> 38
   acggagaggg acataag 17
<210> 39
   <211> 20
   <212> DNA
   <213> Artificial Sequence
<220>
   <223> Subtype-specific oligonucleotide 2b12 immobilized on the biochip
<400> 39
   cataagaaca gaagaatcca 20
<210> 40
   <211> 19
   <212> DNA
   <213> Artificial Sequence
<220>
   <223> Subtype-specific oligonucleotide 2i1 immobilized on the biochip
<400> 40
   tcacygaaag rgacatcag 19
<210> 41
   <211> 16
   <212> DNA
   <213> Artificial Sequence
<220>
   <223> Subtype-specific oligonucleotide 2cj1 immobilized on the biochip
<400> 41
   yagaaccgag gagtcc 16
<210> 42
   <211> 18
   <212> DNA
   <213> Artificial Sequence
<220>
   <223> Subtype-specific oligonucleotide 2k1 immobilized on the biochip
<400> 42
   acggagagrg atatcagg 18
<210> 43
   <211> 18
   <212> DNA
   <213> Artificial sequence
<220>
   <223> Subtype-specific oligonucleotide 211 immobilized on the biochip
<400> 43
   atacggacag aagaatcc 18
<210> 44
   <211> 16
   <212> DNA
   <213> Artificial sequence
<220>
   <223> Subtype-specific oligonucleotide 2m1 immobilized on the biochip
<400> 44
   gggacatycg artcga 16
<210> 45
   <211> 18
   <212> DNA
   <213> Artificial Sequence
<220>
   <223> Subtype-specific oligonucleotide 2a2 immobilized on the biochip
<400> 45
   tacactcgct gactgaga 18
<210> 46
   <211> 18
   <212> DNA
   <213> Artificial Sequence
<220>
   <223> Subtype-specific oligonucleotide 2b2 immobilized on the biochip
<400> 46
   tacactcgct cactgaga 18
<210> 47
   <211> 20
   <212> DNA
   <213> Artificial Sequence
<220>
   <223> subtype-specific oligonucleotide 2c2 immobilized on the biochip
<400> 47
   atacactcac tgactgagag 20
<210> 48
   <211> 18
   <212> DNA
   <213> Artificial Sequence
<220>
   <223> Subtype-specific oligonucleotide 2d immobilized on the biochip
<400> 48
   ctcactgact gagaggct 18
<210> 49
   <211> 21
   <212> DNA
   <213> Artificial Sequence
<220>
   <223> subtype-specific oligonucleotide 2kc2 immobilized on the biochip
<220>
   <221> misc_feature
   <222> (10)..(10)
   <223> n is any of a, g, c, and t
<400> 49
   acactcactn actgagagac t 21
<210> 50
   <211> 20
   <212> DNA
   <213> Artificial Sequence
<220>
   <223> Subtype-specific oligonucleotide 2i2 immobilized on the biochip
<400> 50
   tacactcact ractgagagg 20
<210> 51
   <211> 20
   <212> DNA
   <213> Artificial sequence
<220>
   <223> Subtype-specific oligonucleotide 2j2 immobilized on the biochip
<400> 51
   catacattca ctcactgaga 20
<210> 52
   <211> 20
   <212> DNA
   <213> Artificial Sequence
<220>
   <223> Subtype-specific oligonucleotide 212 immobilized on the biochip
<400> 52
   atyaaatcac tgacagagag 20
<210> 53
   <211> 19
   <212> DNA
   <213> Artificial Sequence
<220>
   <223> subtype-specific oligonucleotide 2m2 immobilized on the biochip
<400> 53
   atacactcay tgaccgaga 19
<210> 54
   <211> 14
   <212> DNA
   <213> Artificial Sequence
<220>
   <223> Subtype-specific oligonucleotide 2a31 immobilized on the biochip
<400> 54
   aaagcyctag cggc 14
<210> 55
   <211> 15
   <212> DNA
   <213> Artificial Sequence
<220>
   <223> Subtype-specific oligonucleotide 2a32 vimmobilized on the biochip
<400> 55
   cyctagcggc ttgya 15
<210> 56
   <211> 14
   <212> DNA
   <213> Artificial Sequence
<220>
   <223> Subtype-specific oligonucleotide 2b31 immobilized on the biochip
<400> 56
   aaagcccttg crgc 14
<210> 57
   <211> 13
   <212> DNA
   <213> Artificial sequence
<220>
   <223> Subtype-specific oligonucleotide 2b32 immobilized on the biochip
<400> 57
   aagccctcgc rgc 13
<210> 58
   <211> 13
   <212> DNA
   <213> Artificial sequence
<220>
   <223> Subtype-specific oligonucleotide 2c3 immobilized on the biochip
<400> 58
   aagccagrgc ggc 13
<210> 59
   <211> 13
   <212> DNA
   <213> Artificial sequence
<220>
   <223> Subtype-specific oligonucleotide 2d3 immobilized on the biochip
<220>
   <221> misc_feature
   <222> (2)..(2)
   <223> n is any of a, g, c, and t
<400> 59
   cnrrrgcagc ctg 13
<210> 60
   <211> 13
   <212> DNA
   <213> Artificial sequence
<220>
   <223> Subtype-specific oligonucleotide 2i3 immobilized on the biochip
<400> 60
   gcycaagcgg cct 13
<210> 61
   <211> 12
   <212> DNA
   <213> Artificial Sequence
<220>
   <223> subtype-specific oligonucleotide 2k3 immobilized on the biochip
<400> 61
   aggccctggc gg 12
<210> 62
   <211> 14
   <212> DNA
   <213> Artificial sequence
<220>
   <223> subtype-specific oligonucleotide 2m3 immobilized on the biochip
<400> 62
   aagcccaagc agcc 14
<210> 63
   <211> 17
   <212> DNA
   <213> Artificial sequence
<220>
   <223> subtype-specific oligonucleotide 3a1 immobilized on the biochip
<400> 63
   acatcagggt ggaagag 17
<210> 64
   <211> 16
   <212> DNA
   <213> Artificial Sequence
<220>
   <223> Subtype-specific oligonucleotide 3b1 immobilized on the biochip
<400> 64
   catcaggacg gaggag 16
<210> 65
   <211> 13
   <212> DNA
   <213> Artificial Sequence
<220>
   <223> Subtype-specific oligonucleotide 3a3 immobilized on the biochip
<400> 65
   aaggccacrg cgg 13
<210> 66
   <211> 14
   <212> DNA
   <213> Artificial Sequence
<220>
   <223> Subtype-specific oligonucleotide 3b3 immobilized on the biochip
<400> 66
   aaggccactg cvgc 14
<210> 67
   <211> 14
   <212> DNA
   <213> Artificial Sequence
<220>
   <223> subtype-specific oligonucleotide 3k3 immobilized on the biochip
<400> 67
   aagcaaaggc agcc 14
<210> 68
   <211> 16
   <212> DNA
   <213> Artificial sequence
<220>
   <223> Subtype-specific oligonucleotide 3a2 immobilized on the biochip
<400> 68
   atctcctccc tcacgg 16
<210> 69
   <211> 16
   <212> DNA
   <213> Artificial Sequence
<220>
   <223> Subtype-specific oligonucleotide 3b2 immobilized on the biochip
<400> 69
   atcagcgctc tcacrg 16
<210> 70
   <211> 18
   <212> DNA
   <213> Artificial Sequence
<220>
   <223> Subtype-specific oligonucleotide 3k2 immobilized on the biochip
<400> 70
   tgataacttc actcacgg 18
<210> 71
   <211> 17
   <212> DNA
   <213> Artificial sequence
<220>
   <223> subtype-specific oligonucleotide 4ac1 immobilized on the biochip
<400> 71
   aaccgaaaag gacatca 17
<210> 72
   <211> 18
   <212> DNA
   <213> Artificial Sequence
<220>
   <223> subtype-specific oligonucleotide 4df1 immobilized on the biochip
<400> 72
   tracygaaag agacatca 18
<210> 73
   <211> 18
   <212> DNA
   <213> Artificial Sequence
<220>
   <223> Subtype-specific oligonucleotide 4hk1 immobilized on the biochip
<400> 73
   tgactgaaag ggacatca 18
<210> 74
   <211> 17
   <212> DNA
   <213> Artificial sequence
<220>
   <223> subtype-specific oligonucleotide 4i1 immobilized on the biochip
<400> 74
   cgtgacggag agagaca 17
<210> 75
   <211> 19
   <212> DNA
   <213> Artificial sequence
<220>
   <223> subtype-specific oligonucleotide 4n1 immobilized on the biochip
<400> 75
   actgtgactg agaaagaca 19
<210> 76
   <211> 17
   <212> DNA
   <213> Artificial Sequence
<220>
   <223> Subtype-specific oligonucleotide 4p1 immobilized on the biochip
<400> 76
   ccgtgactga gaaggac 17
<210> 77
   <211> 17
   <212> DNA
   <213> Artificial sequence
<220>
   <223> subtype-specific oligonucleotide 4r1 immobilized on the biochip
<400> 77
   gtcaccgaaa rrgacat 17
<210> 78
   <211> 16
   <212> DNA
   <213> Artificial sequence
<220>
   <223> subtype-specific oligonucleotide 4a21 immobilized on the biochip
<400> 78
   gttattgcyg ccctca 16
<210> 79
   <211> 15
   <212> DNA
   <213> Artificial sequence
<220>
   <223> Subtype-specific oligonucleotide 4dp2 immobilized on the biochip
<400> 79
   ggtgatatcc gccct 15
<210> 80
   <211> 17
   <212> DNA
   <213> Artificial sequence
<220>
   <223> Subtype-specific oligonucleotide 4a22 immobilized on the biochip
<400> 80
   gcaaagtcat caccgcc 17
<210> 81
   <211> 17
   <212> DNA
   <213> Artificial Sequence
<220>
   <223> subtype-specific oligonucleotide 4c2 immobilized on the biochip
<400> 81
   acygccctaa cagagag 17
<210> 82
   <211> 16
   <212> DNA
   <213> Artificial Sequence
<220>
   <223> subtype-specific oligonucleotide 4f2 immobilized on the biochip
<400> 82
   aggtratatc cgccct 16
<210> 83
   <211> 15
   <212> DNA
   <213> Artificial Sequence
<220>
   <223> Subtype-specific oligonucleotide 4i2 immobilized on the biochip
<400> 83
   aggtcatcaa mgccc 15
<210> 84
   <211> 17
   <212> DNA
   <213> Artificial Sequence
<220>
   <223> subtype-specific oligonucleotide 4k2 immobilized on the biochip
<400> 84
   araccratat ccgccct 17
<210> 85
   <211> 16
   <212> DNA
   <213> Artificial Sequence
<220>
   <223> subtype-specific oligonucleotide 4n2 immobilized on the biochip
<400> 85
   gyyataaccg ccctca 16
<210> 86
   <211> 15
   <212> DNA
   <213> Artificial Sequence
<220>
   <223> subtype-specific oligonucleotide 4o2 immobilized on the biochip
<400> 86
   cgcccttacr gagag 15
<210> 87
   <211> 14
   <212> DNA
   <213> Artificial sequence
<220>
   <223> subtype-specific oligonucleotide 4r2 immobilized on the biochip
<400> 87
   aaggccataa ccgc 14
<210> 88
   <211> 17
   <212> DNA
   <213> Artificial Sequence
<220>
   <223> Subtype-specific oligonucleotide 4t2 immobilized on the biochip
<400> 88
   ggtratayca gccctca 17
<210> 89
   <211> 15
   <212> DNA
   <213> Artificial sequence
<220>
   <223> Subtype-specific oligonucleotide 4a3 immobilized on the biochip
<400> 89
   caaagccaca gccgc 15
<210> 90
   <211> 14
   <212> DNA
   <213> Artificial sequence
<220>
   <223> Subtype-specific oligonucleotide 4c3 immobilized on the biochip
<400> 90
   aaagcctmag ccgc 14
<210> 91
   <211> 15
   <212> DNA
   <213> Artificial Sequence
<220>
   <223> Subtype-specific oligonucleotide 4d3 immobilized on the biochip
<400> 91
   taaggccagc gcagc 15
<210> 92
   <211> 15
   <212> DNA
   <213> Artificial Sequence
<220>
   <223> subtype-specific oligonucleotide 4f3 immobilized on the biochip
<400> 92
   yaaggcyacm gcggc 15
<210> 93
   <211> 15
   <212> DNA
   <213> Artificial sequence
<220>
   <223> subtype-specific oligonucleotide 4h3 immobilized on the biochip
<400> 93
   argccacrgc arcca 15
<210> 94
   <211> 15
   <212> DNA
   <213> Artificial sequence
<220>
   <223> subtype-specific oligonucleotide 4k3 immobilized on the biochip
<400> 94
   ttaaggcygy cgcag 15
<210> 95
   <211> 15
   <212> DNA
   <213> Artificial sequence
<220>
   <223> Subtype-specific oligonucleotide 4on3 immobilized on the biochip
<400> 95
   aagaccacrg ccgcc 15
<210> 96
   <211> 15
   <212> DNA
   <213> Artificial Sequence
<220>
   <223> Subtype-specific oligonucleotide 4i3 immobilized on the biochip
<400> 96
   cctcaargcc acagc 15
<210> 97
   <211> 15
   <212> DNA
   <213> Artificial Sequence
<220>
   <223> Subtype-specific oligonucleotide 4p3 immobilized on the biochip
<400> 97
   yaaggcaaca gcagc 15
<210> 98
   <211> 16
   <212> DNA
   <213> Artificial sequence
<220>
   <223> Subtype-specific oligonucleotide 4r3 immobilized on the biochip
<400> 98
   aaaaccacgg crgcca 16
<210> 99
   <211> 17
   <212> DNA
   <213> Artificial sequence
<220>
   <223> subtype-specific oligonucleotide 4a4 immobilized on the biochip
<400> 99
   tgtgggtatc ggagatg 17
<210> 100
   <211> 15
   <212> DNA
   <213> Artificial Sequence
<220>
   <223> Subtype-specific oligonucleotide 4c4 immobilized on the biochip
<400> 100
   cgggtatcgc agatg 15
<210> 101
   <211> 15
   <212> DNA
   <213> Artificial sequence
<220>
   <223> Subtype-specific oligonucleotide 4d4 immobilized on the biochip
<400> 101
   cggractcga cggtg 15
<210> 102
   <211> 16
   <212> DNA
   <213> Artificial Sequence
<220>
   <223> Subtype-specific oligonucleotide 4f4 immobilized on the biochip
<400> 102
   ygggtaccgt agatgc 16
<210> 103
   <211> 14
   <212> DNA
   <213> Artificial Sequence
<220>
   <223> Subtype-specific oligonucleotide 4h4 immobilized on the biochip
<400> 103
   cggghttcgg aggt 14
<210> 104
   <211> 16
   <212> DNA
   <213> Artificial Sequence
<220>
   <223> subtype-specific oligonucleotide 4i4 immobilized on the biochip
<400> 104
   gtggcatccg tagatg 16
<210> 105
   <211> 16
   <212> DNA
   <213> Artificial Sequence
<220>
   <223> Subtype-specific oligonucleotide 4k4 immobilized on the biochip
<400> 105
   gcgggtatcg vaggtg 16
<210> 106
   <211> 14
   <212> DNA
   <213> Artificial Sequence
<220>
   <223> Subtype-specific oligonucleotide 4o4 immobilized on the biochip
<400> 106
   gccagcggag atgc 14
<210> 107
   <211> 16
   <212> DNA
   <213> Artificial Sequence
<220>
   <223> Subtype-specific oligonucleotide 4pt4 immobilized on the biochip
<400> 107
   cggtgtbcgy aggtgc 16
<210> 108
   <211> 16
   <212> DNA
   <213> Artificial sequence
<220>
   <223> Subtype-specific oligonucleotide 4r4 immobilized on the biochip
<400> 108
   cggttatcgg agatgc 16
<210> 109
   <211> 17
   <212> DNA
   <213> Artificial sequence
<220>
   <223> Subtype-specific oligonucleotide 5a immobilized on the biochip
<400> 109
   gyratacggt cactcac 17
<210> 110
   <211> 17
   <212> DNA
   <213> Artificial Sequence
<220>
   <223> subtype-specific oligonucleotide 6a1 immobilized on the biochip
<400> 110
   cggactgaga acgacat 17
<210> 111
   <211> 18
   <212> DNA
   <213> Artificial sequence
<220>
   <223> Subtype-specific oligonucleotide 6b1 immobilized on the biochip
<400> 111
   cgaactgaag aggacatc 18
<210> 112
   <211> 17
   <212> DNA
   <213> Artificial sequence
<220>
   <223> Subtype-specific oligonucleotide 6d1 immobilized on the biochip
<400> 112
   cggactgagg aggacat 17
<210> 113
   <211> 17
   <212> DNA
   <213> Artificial sequence
<220>
   <223> Subtype-specific oligonucleotide 6g1 immobilized on the biochip
<400> 113
   cggacagagg agtcyat 17
<210> 114
   <211> 17
   <212> DNA
   <213> Artificial Sequence
<220>
   <223> Subtype-specific oligonucleotide 6h1 immobilized on the biochip
<400> 114
   cgcacagaac aagacat 17
<210> 115
   <211> 16
   <212> DNA
   <213> Artificial Sequence
<220>
   <223> Subtype-specific oligonucleotide 6k1 immobilized on the biochip
<400> 115
   actgagcggg atgtct 16
<210> 116
   <211> 13
   <212> DNA
   <213> Artificial Sequence
<220>
   <223> Subtype-specific oligonucleotide 6a3 immobilized on the biochip
<400> 116
   gcacaggccg cct 13
<210> 117
   <211> 13
   <212> DNA
   <213> Artificial Sequence
<220>
   <223> subtype-specific oligonucleotide 6b3 immobilized on the biochip
<400> 117
   gcacaggcgg cgt 13
<210> 118
   <211> 13
   <212> DNA
   <213> Artificial Sequence
<220>
   <223> subtype-specific oligonucleotide 6d3 immobilized on the biochip
<400> 118
   aggcgcaagc agc 13
<210> 119
   <211> 12
   <212> DNA
   <213> Artificial sequence
<220>
   <223> Subtype-specific oligonucleotide 6g3 immobilized on the biochip
<400> 119
   aggccayggc gg 12
<210> 120
   <211> 12
   <212> DNA
   <213> Artificial Sequence
<220>
   <223> subtype-specific oligonucleotide 6h3 immobilized on the biochip
<400> 120
   gcaaccgccg ct 12
<210> 121
   <211> 23
   <212> DNA
   <213> Artificial sequence
<220>
   <223> Forward primer Pr3_f for PCR amplification of the NS5b region fra gment
<400> 121
   tatgayaccc gctgytttga ctc 23
<210> 122
   <211> 30
   <212> DNA
   <213> Artificial sequence
<220>
   <223> Reverse primer Pr2_r for PCR amplification of the NS5b region fr agment
<400> 122
   ggcggaattc ctggtcatag cctccgtgaa 30
<210> 123
   <211> 18
   <212> DNA
   <213> Artificial Sequence
<220>
   <223> Reverse primer Pr5_r for PCR amplification of the NS5b region fr agment
<400> 123
   gctagtcata gcctccgt 18

## Description

The present invention relates to molecular biology, virology and medicine and deals with a method of identification of a genotype and subtype of Hepatitis C virus (HCV) on the basis of the analysis of an HCV genome NS5B region using a differentiating biochip.

HCV is related to the *Flaviviridae* RNA-containing virus family and causes an infectious process with the most frequent complication of cirrhosis and hepatocarcinoma (Surveillance. Hepatitis. CDC Report Nº61; Younossi Z, Kallman J, Kincaid J. The effects of HCV infection and management on health-related quality of life. Hepatology. 2007 Mar; 45(3): 806-16). More than 170 million people on the planet are afflicted by this disease, and the number of the affected is on the increase. There are about 1.5 million hepatocarcinoma cases world-wide caused by HCV infection. Said disease-related loss in the USA alone are 200 mln $ -- an yearly estimate.

A contemporary wide-spread trend in HCV treatment is the use of combination therapy comprising co-injection of megadoses of interferon with a cocktail containing both common antiviral preparations and one or two inhibitors of HCV replication (specific protease-helicase and/or RNA-polymerase inhibitors) (Toniutto P, Fabris C, Bitetto D, Fomasiere E, Rapetti R, Pirisi M. Valopicitabine dihydrochloride; a specific polymerase inhibitor of Hepatitis C virus. Curr Opin Investig Drugs. 2007 Feb; 8(2):150-8; Johnson CL, Owen DM, Gale M Jr. Functional and therapeutic analysis of Hepatitis C virus NS3. 4A protease control of antiviral immune defense. J Biol Chem. 2007 Apr; 282(14): 10792-803). These cocktails increase the percentage of recovery, however inevitably leading to the formation of adaptive, inhibitor-resistant HCV mutants.

The identification of a genotype and subtype of an HCV specimen is of substantive importance for the purpose of detecting treatment response, evaluating duration and efficacy of antiviral therapy and establishing a route of virus propagation.

To define HCV genotypes and subtypes, methods currently used are as follows:

### I. Direct determination of a nucleotide sequence (Sequencing) of an HCV 5'-noncoding region with the subsequent analysis of a determined sequence as compared to the available database to determine the attribution of studied HCV sample to a certain genotype and subtype (kit 'THUGENE HCV 5' NC' (Bayer HealthCare LLC, USA)):

Jeffrey J. Germer, David W. Majewski, Michael Rosser, Amber Thompson, P. Shawn Mitchell, Thomas F. Smith, Slava Elagin, and Joseph D.C. Yao. 2003. Evaluation of the 'THUGENE HCV 5' NC Genotyping Kit with the New GeneLibrarian Module 3.1.2 for Genotyping of Hepatitis C virus from Clinical Specimens. J Clin Microbiol, Vol. 41, No. 10, p. 4855-4857.

### II. Line Probe assay (LiPA):

Zheng X, Pang M, Chan A, Roberto A, Warner D, Yen-Lieberman B.2003. Direct comparison of Hepatitis C virus genotypes tested by INNO-LiPA HCV II and TRUGENE HCV genotyping methods. J Clin Virol. Oct; 28(2):214-6;

Verbeeck J, Maes P, Wollants E, Van der Merwe S, Song E, Nevens F, Van Ranst M.2005. Use of a commercially available line probe assay for genotyping of Hepatitis C virus 5a strains. J Clin Microbiol. Dec; 43(12): 6117-9.

### III. Method of extension of genotype-specific primer (Primer-specific extension analysis):

Antonishyn NA, Ast VM, McDonald RR, Chaudhary RK, Lin L, Andonov AP, Horsman GB. 2005. Rapid genotyping of Hepatitis C virus by primer-specific extension analysis. J Clim Microbiol. Oct; 43(10): 5158-63.

### IV. Mass-spectrometry methods (Matrix-assisted laser desorption ionization-time of flight (MALDI mass spectrometry)):

Ilina EN, Malakhova MV, Generozov EV, Nikolaev EN, Govorun VM. 2005. Matrix-assisted laser desorption ionization-time of flight (mass spectrometry) for Hepatitis C virus genotyping. J Clin Microbiol. Junl 43(6):2810-5.

### V. Methods of enzyme immunoassay (Serotyping of Hepatitis C virus):

Elsawy EM, Sobh MA, El-Chenawi FA, Hassan IM, Shehab El-Din AB, Ghoneim MA 2005. Serotyping of Hepatitis C virus in hemodialysis patients: comparison with a standardized genotyping assay Diagn Microbiol Infect Dis. Feb; 51(2): 91-4.

### VI. Heteroduplex analysis using capillary electrophoresis (heteroduplex mobility analysis using temperature gradient capillary electrophoresis):

Margraf RL, Erali M, Liew M, Wittwer CT. 2004. Genotyping Hepatitis C virus by heteroduplex mobility analysis using temperature gradient capillary electrophoresis J Clin Microbiol. 2004 Oct; 42(10):4545-51.

### VII. Method of invasive probes (Invader Assay):

Germer JJ, Majewski DW, Yung B, Mitchell PS, Yao JD. 2006. Evaluation of the invader assay for genotyping Hepatitis C virus. J Clin Microbiol. Feb; 44(2): 318-23.

### VIII. Method of nested PCR with subsequent specific-structural restriction (Nested restriction site-specific PCR):

Krekulova L, Rehak V, Wakil AE, Harris E, Riley LW. 2001. Nested restriction site-specific PCR to detect and type Hepatitis C virus (HCV): a rapid method to distinguish HCV subtype 1b from other genotypes. J Clin Microbiol. May; 39(5): 1774-80.

IX. High-performance liquid chromatography (denaturing high-performance liquid chromatography):

Liew M, Erali M, Page S, Hillyard D, Wittwer C. 2004 Hepatitis C genotyping by denaturing high-performance liquid chromatography. J Clin Microbiol. Jan; 42(1): 158-63.

X. Transcription mediated amplification in conjunction with probe method (transcription-mediated amplification in conjunction with the line probe assay):

Comanor L, Elkin C, Leung K, Krajden M, Kronquist K, Nicolas K, Horansky E, deMedina M, Kittichai P, Sablon E, Ziermann R, Sherlock C. 2003 Successful HCV genotyping of previously failed and low viral load specimens using an HCV RNA qualitative assay based on transcription-mediated amplification in conjunction with the line probe assay. J Clin Virol. Sep; 28(1): 14-26.

### XI. Real-time PCR followed by melting curve analysis (Melting curve analysis):

Doris M. Haverstick, Grant C. Bullock, and David E. Bruns. 2004. Genotyping of Hepatitis C virus by Melting Curve Analysis: Analytical Characteristics and Performance, Clinical Chemistry 50, No. 12, p. 2405-2407.

### XII. Dirfect determination of a nucleotide sequence (Sequencing) of an HCV NS5B region followed by the constructing a phylogenetic tree and defining a genotype and subtype of the specimen assayed, on the basis of localization of the sequence analyzed in one of the clusters of the tree derived (NS5B sequencing followed by phylogenetic analysis):

K. Sandres-Saune, P.Deny, C. Pasquier, V. Thibaut, G. Duverlie, J. Izopet. 2003. Determining hepatitis C genotype by analyzing the sequence of the NS5B region. Journal of Virological Methods Vol. 109 pp 187-193;

Laperche S, Lunel F, Izopet J, Alain S, Deny P, Duverlie G, Gaudy C, Pawlotsky JM, Plantier JC, Pozzetto B, Thibault V, Tosetti F, Lefrere JJ. 2005. Comparison of Hepatitis C virus NS5B and 5' noncoding gene sequencing methods in a multicenter study. J. Clin Microbiol. Feb; 43(2): 733-9;

Hnatyszyn, J., Beld M., Gualbertus Hubertus M., Guettouche T., Gouw R., Van Der Meer, C., Beatrijs Maria. (Bayer Healthcare LLC). Methods and reagents for genotyping HCV. WO/2007/076493. International Application No PCT/US2006/062582. Publication Date: 05.07.2007.

Methods (I-IV, VI-XI) are based on the analysis of genotype - and subtype specific sequences of an HCV 5'-noncoding region (5' NC). The analysis of the 5' NC region makes it possible to clearly identify all the six HCV genotypes, albeit showing low efficiency (less than 70%) with reference to differentiation of subtypes belonging to genotype 1, specifically a subtype 1b that is most virulent and resistant to ribavirin/interferon treatment (K.Sandres-Saune, P. Deny, C.Pasquier, V.Thibaut, G. Duverlie, J. Izopet. 2003. Determining hepatitis C genotype by analyzing the sequence of the NS5B region. Journal of Virological Methods Vol. 109 pp 187-193; Laperche S, Lunel F, Izopet J. Alain S, Deny P, Duverlie G, Gaudy C, Pawlotsky JM, Plantier JC, Pozzetto B, Thibault V, Tosetti F, Lefrere JJ. 2005. Comparison of Hepatitis C virus NS5B and 5' noncoding gene sequencing methods in a multicenter study. J Clin Microbiol. Feb; 43(2): 733-9; Cantaloube JF, Laperche S, Gallian P, Bouchardeau F, de Lamballerie X, de Micco P. Analysis of the 5' noncoding region versus the NS5B region in genotyping Hepatitis C virus isolates from blood donors in France. J Clin Microbiol. 2006 Jun; 44(6):2051-6; Murphy DG, Villems B, Deschenes M, Hilzenrat N, Mousseau R, Sabbah S.2007. Use of Sequence Analysis of the NS5B Region for Routine Genotyping of Hepatitis C virus with Reference to C/E1 and 5'UTR Sequences. J Clin Microbiol. Feb. 7).

At present only the analysis of a NS5B region permits identifying a subtype 1b with the specificity approaching 100%. Moreover, the investigation of sequences of the given region enables detection of a number of subtypes much greater than those in the analysis of the 5' NC sequences (Thomas F, Nicot F, Sandres-Saune K, Dubois M, Legrand-Abravanel F, Alric L, Peron JM, Pasquier C, Izopet J. 2007. Genetic diversity of HCV genotype 2 strains in south western France. J Med Viol. Jan; 79(1): 26:34; Nicot F, Legrand-Abravanel F, Sandres-Saune K, Boulestin A, Dubois M, Alric L, Vinel JP, Pasquier C, Izopet J. 2005. Heterogeneity of Hepatitis C virus genotype 4 strains circulating in south-western France. J Gen Virol Jan; 86(Pt 1): 107-14).

Thus, the analysis of NS5B region sequence is now necessary for the identification of the genotype and subtype of an HCV specimen for the purpose of detecting a treatment response, evaluating duration and efficiency of antiviral therapy, establishing an infection route (Laperche S, Saune K, Deny P, Duverlie G, Alan S, Chaix ML, Gaudy C, Lunel F, Pawlotsky JM, Payan C, Pozzetto B, Tamalet C, Thibault V, Vallet S, Bouchardeau F, Izopet J, Lefrere JJ. 2006. Unique NS5B Hepatitis C virus gene sequence consensus database is essential for standardization of genotype determinations in multicenter epidemiological studies. J Clin Microbiol Feb; 44(2):614-6; Kuiken C, Yusim K, Boykin L, Richardson R. 2005. The Los Alamos hepatitis C sequence database. Bioinformatics Feb 1; 21(3):379-84).

A method for sequencing an HCV NS5B region followed by a phylogenetic assay (X11) calls for amplification and sequencing reactions, a further purification of reaction products subsequent to each of the above-mentioned steps and the following automatic sequencer analysis. More, the following analysis of chromatograms, constructing the multiple alignment and building phylogenetic trees exact the highest requirements for the skill of laboratory personnel, a factor that is a bar to the comprehensive use of the given approach for the analysis of a current of clinical specimens in the conditions of an ordinary diagnostic laboratory.

A method for detecting serotypes through the use of variants of an enzyme immunoassay (V) permits indentifying only a restricted number of genotypes and subtypes (1a, 1b, 2a, 2b, 3a, and 4a) and calls for the presence of highly purified monoclonal antibodies for each and every serotype.

Likewise, above-listed methods of identification of genotypes and subtypes have the following drawbacks:
- commercial kit INNO-LiPA (II) and its use in conjunction with transcription-mediated amplification (X) is distinguished for high cost and identifies a limited number of subtypes;
- method based on the use of genotype-specific primers (III) requires independent reactions by the number of genotypes (i.e. no less than 6) to detect one genotype only;
- PCR-heteroduplex analysis using capillary electrophoresis (VI) and a method of nested PCR with subsequent specific-structural restriction (VIII) are much labour- and time-consuming and require standards for each genotype and/or subtype to be determined;
- method of invasive probes (VII) identifies only a genotype and does not applicable for subtype determination, which is a serious restriction for using same in clinical practice where it is necessary to detect HCV drug-resistant varieties (at least 1b and 4d) to evaluate the efficiency and duration of therapy;
- methods of mass spectrometry (IV) and HPLC (IX) call for availability of expensive equipment, additional steps for preparing a specimen for assay and identify the limited number of subtypes;
- Real-time PCR (XI) detects the presence only of the most wide-spread genotypes and subtypes and is very expensive for routine analysis.

It is hence only logical to see that in the present field there is an urgent need of developing a method for identifying an HCV genotype and subtype that can be used to advantage against a background of solutions known from state of the art and is distinguished for the simplicity of conduct of an analysis, high specificity and information content with respect to the number of identifiable genotypes and subtypes and also low cost.

As a result of comprehensive research, the authors of the present invention have discovered that the task of working out a method for the identification of an HCV genotype and subtype can successfully be solved through the use of biological chips (microchips) for the analysis of an HCV genome NS5B region.

A method for the identification of a genotype and a subtype of Hepatitis C virus (HCV) on the basis of the analysis of an HCV genome NS5B region on biochips is advantageously distinguished from methods known from state of the art adapted to detect all six genotypes (1-6) and 36 subtypes of Hepatitis C virus (1a-1e, 2a, 2b, 2c, 2d, 2i, 2j, 2k, 21, 2m, 3a, 3b, 3k, 4a, 4c, 4d, 4f, 4h, 4i, 4k, 4n, 4o, 4p, 4r, 4t, 5a, 6a, 6b, 6d, 6g, 6h, 6k) in clinical specimens showing a specificity approximating 100% due to the analysis of the NS5B region sequence; and also low cost and little time required for obtaining results. The method does not call for expensive equipment and highly skilled personnel. Data provided by a method of hybridization on the biochips can be used for evaluating and predicting severity of a disease (acute/chronic cirrhosis, a likelihood of liver cancer development), determining a therapeutic dosage for medicaments and duration of a course of therapy as well as for epidemiological genotyping.

In its first aspect, the present invention provides for a method of identification of an HCV genotype and subtype on the basis of the analysis of an HCV genome NS5B region using an oligonucleotide biochip. The method of the present invention is based on a two-stage PCR for obtaining a fluorescent labeled, predominately single-stranded, fragment of the NS5B region followed by hybridization of this fragment on the biochip comprising a set of specific discriminating oligonucleotides complementary to the genotype- and subtype-sequences of NS5B region. The method includes the following steps:
(a) reverse transcription combined with PCR (RT-PCR) using a viral RNA as a template and a first pair of primers specific for an NS5B region fragment;
(b) - asymmetric amplification of the NS5B region fragment using as a template the RT-PCR product produced in step (a), a second pair of specific primers and a mixture of four deoxynucleoside triphosphates, wherein one of the said four deoxynucleoside triphosphates is fluorescent labeled, as a substrate, to provide substantially a single-stranded fluorescent labeled fragment;
(c) - providing a biochip for the identification of an HCV genotype and subtype representing a support comprising a set of discrete elements, with a unique oligonucleotide probe immobilized in each of them, having a sequence complementary to the sequence of a single-stranded fragment obtained in step (b) and selected from the group comprising:
   a) corresponding NS5B region fragment sequences specific for each of the HCV genotypes (genotype-specific); and b) corresponding NS5B region fragment sequences specific for each of the HCV subtypes (subtype-specific);
   (d) - hybridization an amplified labeled product from step (b) on a biochip with the formation of duplexes with immobilized probes in conditions providing for a single-nucleotide resolution between the hybridization perfect and imperfect duplexes;
   (d) - registration and interpretation of hybridization results.

In one of its embodiments, a method is characterized in that in step (a) a first pair of specific primers is used whose sequences are set forth in SEQ ID NO: 121 and 122.

In another embodiment, a method is characterized in that in step (b) a second pair of specific primers is used whose sequences are set forth in SEQ ID NO: 121 and 123.

In its further embodiment, a method is characterized in that in step (b) one of the primers of the second pair is used in at least tenfold molar excess relative to a second primer.

In its further embodiment, a method is characterized in that in step (b) the fluorescent labeled deoxynucleoside triphosphate used corresponds to the fluorescent labeled deoxyuridine triphosphate.

In its still further embodiment, a method is characterized in that the biochip is a hydrogel elements-based biochip obtained by the method of chemically or photoinduced copolymerization.

In one more embodiment thereof, a method is characterized in that a biochip comprises a set of immobilized oligonucleotides whose sequences are set forth in SEQ ID NO: 1-120.

According to another embodiment, a method is characterized in that registration of the results of step (d) is performed through the use of a portable analyzer of fluorescence and software, which permits using the software-based processing of signal intensities with the subsequent interpretation of results.

According to still another embodiment, a method is characterized in that interpretation of the registered results of step (d) is performed in two steps: in a first step, signals are analyzed in biochip elements comprising oligonucleotide probes specific for HCV genotypes thereby to identify the genotype of a specimen; analyzed are in case of a genotype being identified in a second step, only biochip elements comprising oligonucleotide probes specific for the subtypes of an identifiable genotype, regardless of the presence of signals in the elements comprising probes specific for the subtypes of other genotypes.

And last but not least, according to yet another embodiment, a method further comprises evaluating and predicting severity of a disease (acute/chronic cirrhosis, a likelihood of liver cancer development), determining a therapeutic dosage of medicaments and duration of therapy and/or epidemiological genotyping on the basis of interpretation of hybridization results.

In its following aspect, the present invention relates to a biochip for the identification of an HCV genotype and subtype, on the basis of NS5B region analysis that represents a support comprising a set of discrete elements, with a unique oligonucleotide probe immobilized in each of them, and the probe sequences are set forth in SEQ ID NO: 1-120.

In another embodiment of the given aspect of the present invention, a biochip is characterized in that it represents a biochip based on hydrogel elements that is obtained by a method of chemically or photoinduced copolymerization.

The following aspect of the present invention is a set of oligonucleotide probes for obtaining a biochip to indentify an HCV genotype and subtype on the basis of NS5B region analysis having the sequences of SEQ ID NO: 1-120.

And last but not least still another aspect of the present invention is a method for designing a set of oligonucleotide probes usable for constructing a biochip of the type used for identifying an HCV genotype and subtype on the basis of analysis of an NS5B region that provides for a separate selection of several discriminating probes for each and every genotype and subtype whose sequences are complementary to the sequences of different segments of an NS5B region fragment as assayed.

Other aspects of the present invention will become clear from the accompanying figures, the claims and a detailed specification.

To gain a better insight into a concept of invention, as being claimed and as set forth in the application, and to demonstrate its characteristic features and advantages there is given a detailed description of invention with reference to the accompanying drawings illustrating a specific embodiment thereof, in which:
Fig. 1 - schematic diagram of the analysis of an HCV NS5B region for identifying an HCV genotype and subtype on a biological microchip.
Fig. 2 - diagram of a selection of oligonucleotides for the identification of genotypes and subtypes on the basis of the analysis of an HCV genome NS5B region fragment.
Fig. 3 - diagram of biochip structure.
Fig. 4A - a fluorescent hybridization pattern obtained on a biochip as a result of analysis of an HCV specimen having a genotype 1, subtype 1a.
Fig. 4B - distribution of normalized signals of biochip elements obtained as the result of the analysis of an HCV specimen having a genotype 1, a subtype 1a.
Fig. 5A - a fluorescent hybridization pattern obtained on a biochip as a result of analysis of an HCV specimen having a genotype 1, subtype 1b.
Fig. 5B - distribution of normalized signals of biochip elements obtained as the result of the analysis of an HCV specimen having a genotype 1, subtype 1b.
Fig. 6A - a fluorescent hybridization pattern obtained on a biochip as a result of analysis of an HCV specimen having a genotype 1, subtype 1e.
Fig. 6B - distribution of normalized signals of biochip elements obtained as the result of the analysis of an HCV specimen having a genotype 1, subtype 1e.
Fig. 7A - a fluorescent hybridization pattern obtained on a biochip as a result of analysis of an HCV specimen having a genotype 2, subtype 2a.
Fig. 7B - distribution of normalized signals of biochip elements obtained as the result of the analysis of an HCV specimen having a genotype 2, subtype 2a.
Fig. 8A - a fluorescent hybridization pattern obtained on a biochip as a result of analysis of an HCV specimen having a genotype 2, subtype 2i.
Fig. 8B - distribution of normalized signals of biochip elements obtained as the result of the analysis of an HCV specimen having a genotype 2, subtype 2i.
Fig. 9A - a fluorescent hybridization pattern obtained on a biochip as a result of analysis of an HCV specimen having a genotype 3, subtype 3a.
Fig. 9B - distribution of normalized signals of biochip elements obtained as the result of the analysis of an HCV specimen having a genotype 3, subtype 3a.
Fig. 10A - a fluorescent hybridization pattern obtained on a biochip as a result of analysis of an HCV specimen having a genotype 4, subtype 4a.
Fig. 10B - distribution of normalized signals of biochip elements obtained as the result of the analysis of an HCV specimen having a genotype 4, subtype 4a.
Fig. 11A - a fluorescent hybridization pattern obtained on a biochip as a result of analysis of an HCV specimen having a genotype 4, subtype 4d.
Fig. 11B - distribution of normalized signals of biochip elements obtained as the result of the analysis of an HCV specimen having a genotype 4, subtype 4d.
Fig. 12A - a fluorescent hybridization pattern obtained on a biochip as a result of analysis of an HCV specimen having a genotype 5, subtype 5a.
Fig. 12B - distribution of normalized signals of biochip elements obtained as the result of the analysis of an HCV specimen having a genotype 5, subtype 5a.
Fig. 13A - a fluorescent hybridization pattern obtained on a biochip as a result of analysis of an HCV specimen having a genotype 6.
Fig. 13B - distribution of normalized signals of biochip elements obtained as the result of the analysis of an HCV specimen having a genotype 6.

The object of the present invention is to develop a method of identifying an HCV genotype and subtype, on the basis of the analysis of NS5B region using biological microchip.

The method envisages the following steps: a reverse transcription procedure combined with a polymerase chain reaction (RT - PCR) for the amplification of NS5B region fragment with the use of viral RNA isolated from clinical sample such as blood, plasma or liver biopsy material, accumulation of single-stranded fluorescent labeled NS5B fragment with the use of cDNA fragment obtained on RT-PCR step. Also, the method as claimed provides for using an original oligonucleotide biochip with immobilized specific probes, procedures of hybridization, registration and interpretation of results.

### The general concept of identification of genotype and subtype of HCV specimen using biochip.

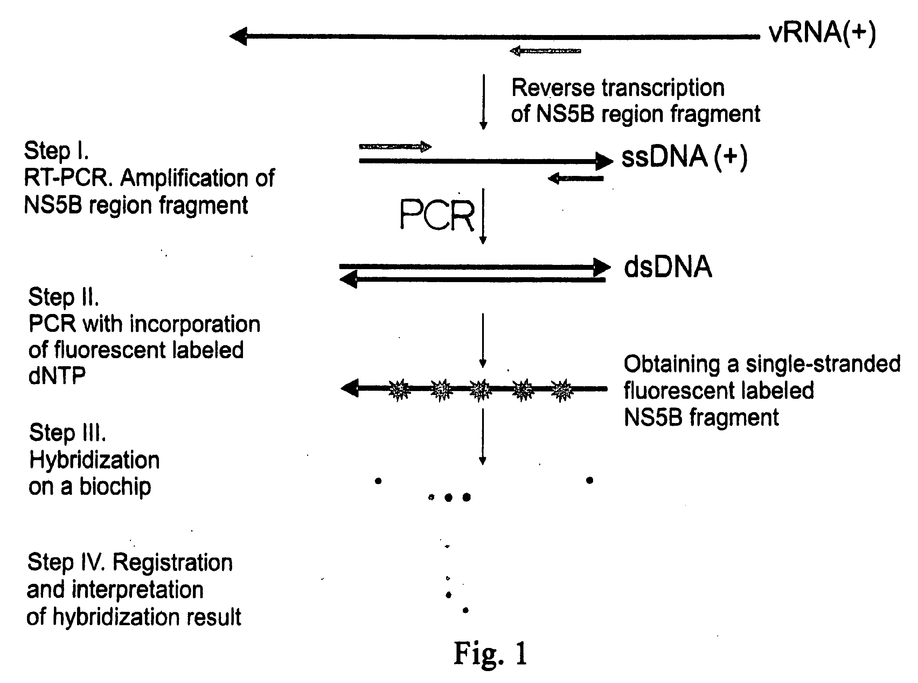

The analysis diagram of the NS5B region fragment for the identification of HCV genotype and subtype using biochip is shown in Fig. 1.

Isolation of HCV RNA from a clinical specimen is carried out through the use of methods known in the given field (for example, Hourfar MK, Michelsen U, Schmidt M, Berger A, Seifried E, Roth WK. High-throughput purification of viral RNA based on novel aqueous chemistry for nucleic acid isolation. Clin Chem. 2005 Jul; 51(7): 1217-22) or any specialized commercially available kit of reagents for isolating RNA from blood, plasma or liver biopsy material, for example, QIAamp DSP Virus Kit (Cat Nº 60704, Qiagen, Germany), MagMAX^{™} AI/ND Viral RNA Isolation Kits (Cat. Nº AM1939, Ambion, USA) or "Kit of reagents for RNA isolation Cat. Nº 05-013, ZAO "DNA technology, Ltd, Russia).

Amplification of an HCV genome NS5B region fragment is carried out in a first step by reverse transcription reaction combined with PCR (RT-PCR). To perform RT-PCR various systems can be used, as shown and described, for example, in Casabianca A., Orlandi C., Fraternale A., Magnani M. A new one-step RT-PCR method for virus quantitation in murine AIDS. 2003 Journal of Virological Methods Vol 110(1), pp. 81-90, and commercially produced kits, e.g., OneStep RT-PCR Kit (Cat. Nº 210210, Qiagen, Germany), Accuscript® High-Fidelity RT-PCR Kit (Cat. Nº 600180, Stratagene, USA) etc.

Primers for performing a first amplification are selected in such a way as to flank the most polymorphic fragment of an NS5B region that allows to differentiate the existing HCV genotypes and subtypes. The NS5B region fragment being amplified is preferred to include HCV genome positions 8256 to 8645 according to Choo, Q.L., K.H. Richman, J.H. Han, K.Berger, C.Lee, C.Dong, C. Gallegos, D. Coit, R. Medina-Selby, P.J.Barr, et al. 1991. Genetic organization and diversity of the Hepatitis C virus. Proc. Natl. Acad. Sci. USA 88: 2451-2455.

Primer sequences are selected in such a way as to perform the effective RNA amplification of the analyzed NS5B region fragment, of any HCV genotype and, accordingly, subtype. For this purpose, the multiple sequence alignment may be constructed using available databases of NS5B region sequences, such as http://www.ncbi.nlm.nih.gov/Genbank/index.html and htttp://hcv.lanl.gov/content/hcv-db. The next step includes the location of the most conservative segments within the analyzed fragment of NS5B region for all HCV genotypes and selection of primers specific to segments concerned. Using the specialized software, for example, Oligo v. 6.3 (Molecular Biology Insights Inc., USA) or Fast PCR (http://www.biocenter.helsinki.fi/bi/Programs/fastpcr.htm) or other commercially available programs or programs free accessible over a world wide web network, melting temperatures of primers are calculated and the lengths of primers are varied, thus providing for a spread of the annealing temperatures of the primers inside a pair of not greater than 3-4°C. Also, the sequences are to be avoided which are able to form secondary structures of a hairpin loop type with high melting temperatures. Each and every selected primer should show a unique specificity to the analyzed NS5B region fragment. The specificity of primers is verified with the help of software using a search in the bases of nucleotide sequences by the BLAST algorithm (www.ncbi.nlm.nih.gov/BLAST/). In particular, the sequences, capable of the efficient hybridization (annealing) with the human genome sequences, should be avoided..

In a second step, a single-stranded fluorescent labeled product is predominantly obtained by an asymmetric PCR using with the use of deoxynucleoside triphosphates mix as the substrate wherein one of said deoxynucleoside triphosphates is fluorescent labeled. Generally, the deoxynucleoside triphosphates mix consists of dATP, dGTP, dCTP 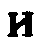 dTTP, and the latter can be replaced with dUTP or a mixture of dTTP/dUTP in any molar proportion. Any one of said deoxynucleoside triphosphates may be fluorescent labeled. Use of a fluorescent labeled deoxyuridine triphosphate is most preferable, which, on the one hand, necessitates the efficient incorporation of the present substrate in the newly synthesized DNA strand during PCR. On the other hand, the application of dUTP-fluorescent labeled conjugates makes it possible to prevent cross-contamination using uracil-DNA-glycosylase enzyme. The latter condition is important for the routine analyses in the clinical laboratory.

The fluorescent dye used can be represented by any fluorescent dye which may chemically be included in the deoxynucleoside triphosphate molecule in such a way as the final conjugate does not hamper the nucleic acids amplification and the subsequent hybridization of the polynucleotide molecule comprising such fluorescent labeled nucleotide residues with immobilized oligonucleotide probes. In the case of a fluorescent labeled deoxyuridine triphosphate, for example, the fluorescent dye can be attached at the 5'-terminal of a dUTP aminoallyl derivative. Examples of such dyes are well known to a person skilled in the art and include fluorescein (TAMRA^{®}, ROX^{®}, JOE^{®}), rhodamine (Texas Red^{®}), polymethine (Cy3^{®}, Cy5^{®}, Cy5.5^{®}, Cy7^{®}) dyes (Ranasinghe R and Brown T). Fluorescence based strategies for genetic analysis. Chem. Commun, 2005, 5487-5502). The fluorescent dyes are commercially available, particularly from the Molecular probes company, USA. The dyes whose excitation spectrum is within a long-wave(Fed) region are most preferable, which permits using inexpensive semiconductor laser as exciting radiation sources for fluorescence excitation.

Fluorescent labeled deoxynucleoside triphosphates can be obtained in laboratory conditions using known methods, such as, for example (Kuwahara M, Nagashima J, Hasegawa M, Tamura T, Kitagata R, Hanawa K, Hososhima S, Kasamatsu T, Ozaki H, Sawai H. Systematic characterization of 2'-deoxynucleoside- 5'-triphosphate analogs as substrates for DNA polymerases by polymerase chain reaction and kinetic studies on enzymatic production of modified DNA. Nucleic Acids Res. 2006 34(19): 5383-94 and are also commercially available, for example, CyDye Fluorescent Nucleotides (Cat. No PA55021, PA55032, PA55026, GE Healthcare, USA).

Primers for the second step of amplification are selected with the requirements set forth above, with the only difference that at least one of the primers is selected inside a PCR fragment from the first step, to enhance reaction specificity. It is hence only logical to see that the resulted PCR fragment will be a product of semi-nested or nested amplification reaction. The length of a second-step amplified fragment is not especially restricted until this enables the efficient hybridization of a fragment with biochip-immobilized probes. In case of biochip with hydrogel elements, the primers for the second amplification step are selected such that the length of an amplified fragment should not exceed 800 nucleotides. The greater length of a PCR product from the second step makes difficult the efficient diffusion of a PCR product as assayed in biochip gel elements during the hybridization, which may result in reducing the number of hybridization duplexes and, consequently, a fluorescent signal fall. On primers design, account should be taken of the fact that single-stranded fluorescent labeled fragment yielded from a second PCR step should be complementary to oligonucleotides immobilized on the biochip. Therefore in each and every pair, the primer added in an excessive amount ("leader primer") is selected from a chain whose sequence is complementary to the sequences of the biochip-immobilized oligonucleotides. That is, should immobilization oligonucleotides be selected from a sense chain, for hybridization duplexes to be formed in biochip elements, there is a need for the primary amplification of an antisense chain and the "leader primer" is thus selected from the chain complementary to a gene sequence (antisense chain), and vice versa.

To provide predominantly a single-chain fluorescent labeled product, a leader primer is added in an excessive molar amount in relation to a second primer. Preferably the molar excess is at least tenfold.

On selection of discriminating oligonucleotides for immobilization on a biochip to take account of the size and complexity of a sequence as assayed and, in particular, the presence of replicas and extended homopolymeric sequences, there is determined a length of the discriminating oligonucleotides that provides their specificity relative to the sequence as assayed.

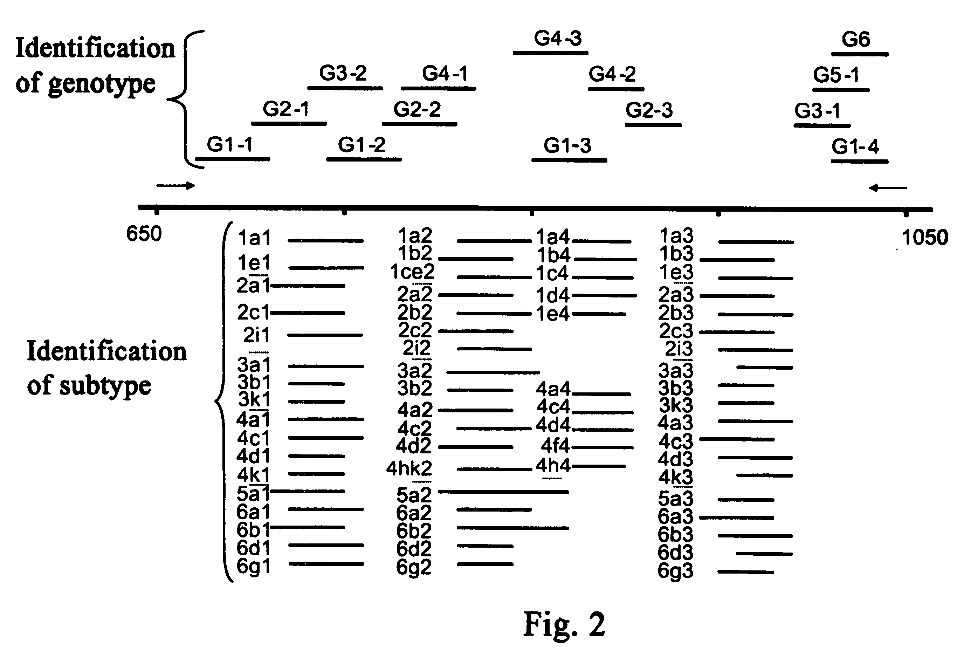

Selection of discriminating oligonucleotides for genotypes and subtypes is carried out in the following manner. Using constructed multiple alignment of NS5B region sequences, the special consensus sequence is generated for each genotype on whose basis are selected unique probes permitting uniquely identifying each and every genotype. Owing to the high variability of an HCV genome, for enhancing the reliability of a method, several discriminating probes for each of the genotypes are selected, if possible, complementary to various segments of the NS5B region fragment as assayed. The consensus sequence is also generated for each subtype followed by the location of segments of the NS5B region fragment which enable to differentiate the maximum number of subtypes inside one genotype. The number of such segments should be enough for providing the reliable identification of each of the subtypes. On the basis of the sequences of the segments selected, probes are constructed for the identification of subtypes, and the sequence of one probe may conform to two or more subtypes simultaneously in the separate differentiating segment of the NS5B region fragment as assayed. The strategy of probes selection for biochip immobilization is schematically shown in Fig. 2.

Using the software, for example, Oligo v. 6.3 (Molecular Biology Insights Inc., USA), melting temperatures of oligonucleotides are calculated and the lengths of probes are varied thereby to provide for a variation of melting temperatures of the oligonucleotides ranging between 2 and 3°C. Oligonucleotides are avoided which are capable of forming secondary structures of a hairpin loop type with high melting temperatures.

Discriminating oligonucleotides are immobilized on a biochip support. The suitable support that might be used to produce the biochip are represented by an activated, say, aminated surface of glass slides (Adessi C., Matton G., Ayala G., Turcatti G., Mermod J., Mayer P., Kawashima E. Solid phase DNA amplification: characterization of primer attachment and amplification mechanisms Nucleic Acids Research. 2000. V.51. 28(20).: E87), plastic wafers (Nikiforov T., Rendle R. Goelet P., Rogers Y., Kotewicz M., Anderson S., Trainor G., Knapp Michael R. Genetic Bit Analysis: a solid phase method for typing single nucleotide polymorphisms. Nucleic Acids Research. 1994. 22(20):4167-75), wafers with polymeric macroporous carriers. such as acrylamide (Timofeev E., Kochetkova S., Mirzabekov A., Florentiev V. Regioselective immobilization of short oligonucleotides to acrylic copolymer gels. Nucleic Acids Res. 1996 24 (16):3142-8, a cepharose (Margulies M, Egholm M, Altman WE, Genome sequencing in microfabricated high-density picolitre reactors. Nature, 2005 437(7057): 376-80) et al. Methods of immobilizing the oligonucleotides on substrates are well known in the given field and comprise:
- chemosorption of oligonucleotides and DNA comprising a thiol group on metals (Mirkin C.A., Letsinger R.L., Mucic R.C. and Storhoff J.J. A DNA-based method for rationally assembling nanoparticles into macroscopic materials. Nature. 1996 Aug 15; 382(6592):607-9);
- covalent binding of modified oligonucleotides with functional groups of a surface based on the reactions of amide bond formation (Healey BG, Matson RS, Walt DR. Fiberoptic DNA sensor array capable of detecting point mutations. Anal Biochem. 1997 251(2): 270-9), ester bond formation (Ghosh SS, Musso GF. Covalent attachment of oligonucleotides to solid supports. Nucleic Acids Res. 1987 15(13):5353-72), carbamimidoyl function (Matson RS, Rampal J, Pentoney SL, Anderson PD, Coassin P. Biopolymer synthesis on polypropylene supports: oligonucleotide arrays. Anal Biochem. 1995 Jan 1; 224(1):110-6), to mention only few;
- photochemically, chemically and electrochemically induced copolymerization of oligonucleotides carrying an unsaturated group with monomers being the basis for a solid phase to be formed (Vasiliskov A.V., Timofeev E.N., Surzhikov S.A., Drobyshev A.L., Shick V.V. and Mirzabekov A.D., Fabrication of microarray of gel-immobilized compounds on a chip by copolymerization. Biotechniques, 1999, 27, 592-606).

In a preferable embodiment of the present invention, use is made of a biochip based on hydrogel elements. Methods for producing such biochips comprise polymerizing amino-modified oligonucleotides to create a covalent bond with gel monomers in suitable conditions (pH, temperature, composition of polymers and so on) (Rubina AY, Pan'kov SV, Dementieva EI et al. Hydrogel drop microchips with immobilized DNA: properties and methods for large-scale production. Anal Biochem 2004; 325: 92-106). Use of biochips comprising gel elements are most preferable, which are applied to a support dropwise with a dia. of 80 to 300 mcm at an interval of 150 to 500 mcm without using special devices-quartz masks, for example. The support used can be represented by a glass substrate (glass slides or cover glass) as well as more available materials, such as plastic materials. To immobilize the oligonucleotides into gel elements of biochip their copolymerization with main gel components is used. As result of this single-stage reaction the immobilized molecules are irreversibly attached in a covalent manner to some or other monomers of a growing polymeric chain and uniformly distributed within the entire volume of a gel with a high yield (about 50% for oligonucleotides) (Rubina AY, Pan'kov SV, Dementieva EI et al. Hydrogel drop microchips with immobilized DNA: properties and methods for large-scale production. Anal Biochem 2004; 325: 92-106). The concentration of immobilized oligonucleotide probes can be judged by staining the gel elements of the biochip with a dye showing a low specificity to a DNA nucleotide sequence (A.L. Mikheikin, A.V. Choudinov, A.I. Yaroschuk, A.Yu. Roubina, S.V. Pan'kov, A.S. Krylov, A.S. Zasedatelev, A.D. Mirzabekov. The dye showing a low specificity to the DNA nucleodie sequence: use for evaluating the number of oligonucleotides immobilized in the elements of biological microchips. Molecular biology 2003; 37(6): 1061-70).

PCR-products from a second amplification step are hybridized on a biochip with immobilized differentiating oligonucleotides complementary to the consensus sequences of genotypes and subtypes of an NS5B region fragment. Hybridization is carried out in a solution containing a buffer component for maintaining a pH value, a salt for creating ionic strength and a chaotropic (hydrogen-bond destabilizing) agent in a hermetically sealed hybridization chamber at a temperature depending on the melting temperatures of immobilized discriminating oligonucleotides. The hydrogen-bond destabilizing agent that might be used can be represented by, for example, guanidine thiocyanate, urea or formamide. A choice of the most favourable hybridization temperature is made to take account of convenience of the practical use of a system. The discriminating oligonucleotides of the present invention have melting temperatures ranging between 42 and 44°C, which fact allows one to carry out hybridization at 37°C using said chaotropic agent. The temperature of 37°C is suitable in that a majority of clinical laboratories are equipped with thermostats maintaining this temperature.

The DNA fragment, as assayed, forms perfect hybridization duplexes only with adequate (fully complementary) oligonucleotides. With all the remaining oligonucleotides said DNA fragment provides an imperfect duplex. Said perfect and imperfect duplexes are discriminated by comparing the fluorescence intensities of biochip elements wherein the duplexes have formed. The signal strength in the element with the perfect hybridization duplex formed therein (I perf.) is higher than in the element where imperfect duplex (I imperf.) has been formed. Hybridization performed in the most favourable conditions (temperature, the concentration of a chaotropic (hydrogen - bond - destabilizing) agent and hybridization buffer ionic strength) provides a I_{perf·.}/I_{imperf.} ≥ 1.5 ratio between two elements comprising probes belonging to one group and differing by one nucleotide.

Registration of hybridization results on biochips can be performed with the aid of commercial scanning devices - analyzers of biochip fluorescence, for example, GenePix 4000B (Axon Instruments, USA) equipped with the adequate software for calculating the strength of fluorescent signals of the discrete elements of a biochip and their subsequent normalization for a background value, for example, 'GenePix Pro', 'Acuity' (Axon Instruments, USA).

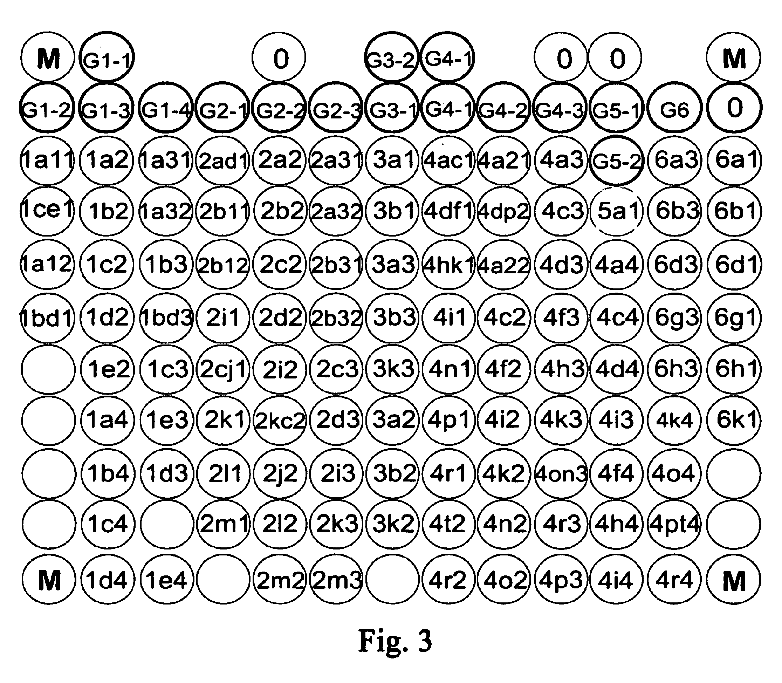

Interpretation of hybridization results can be performed visually through the correlation of the registered fluorescence pattern of a biochip and/or distribution of the signal strength of biochip elements thus obtained to the arrangement of specific discriminating probes in the biochip elements (cf. Fig. 3). Given the distributed signal strength in biochip elements, a maximum signal is detected from among the elements comprising genotype-specific oligonucleotides. A genotype can be identified by providing a biochip element having a maximum fluorescence intensity among the probe-containing elements to determine an HCV genotype. Identification of the subtype of an HCV specimen as assayed can be realized by determining the maximum signals in the elements containing subtype-specific oligonucleotides corresponding to the genotype, as determined, in case of the maximum signals being registered in at least two different elements which contain unique subtype-specific probes.

Interpretation of hybridization results on biochips is preferably performed in the following manner. First step: extraction of valid signals, more exactly the signals in elements, wherein perfect duplexes might be formed, for which purpose the normalized fluorescent strength signals of all biochip elements are classified as to increase and compared with an average signal (I_{ref}) in the elements devoid of any oligonucleotides. The valid signals are those exceeding I_{ref} at least 1.5 times. Second step: starting with an analysis of filtered valid signals Gᵢ in the groups of elements containing genotype-specific oligonucleotides (i - genotype number). A maximum signal is extracted inside each group of elements Gᵢₘₐₓ and compared with each other. If the signal Gᵢₘₐₓ in one group exceeds the maximum signals in the remaining groups more than 1.5 times (a threshold value), a conclusion is drawn on a specimen, as assayed, belonging to the given genotype. If a ratio of signals among Gᵢₘₐₓ does not exceed the threshold value, a conclusion is made on the impossibility to clearly identify a genotype and on the possible presence in the specimen, as assayed, of a mixture of two and more HCV variants with various genotypes. If the signals in genotype-specific- oligonucleotides-containing groups do not undergo primary filtration in relation to the I_{ref}, a conclusion is drawn on low signal strength and the possible absence of an HCV RNA in the specimen as assayed. And no subtype identification is performed whatever.

Thus, given the determined genotype on the basis of a signal Gᵢₘₐₓ, value, a consideration is further given to only the groups of elements comprising oligonucleotides specific for subtypes relating to an identifiable genotype. In accordance with the proposed strategy of selecting probes for the identification of a subtype, the oligonucleotides are combined in groups according to the selected segments of an NTS5B fragment as assayed that permits differentiating the maximum number of the subtypes. And the number of groups is varied from one to four in relation to the degree of homology of the consensus sequences of the NS5B fragment for various subtypes and is dictated by the need for a reliable differentiation of subtypes inside the genotype. On identification of the subtype first picked out are signals inside each group of the elements exceeding the remaining signals of this group at least 1.5 times. This signal is designated as Sᵢₓⱼ (i - genotype number, 'x'-symbol of a subtype according to HCV subtype classification, j - group number). Should two or more elements in the group have signals differing from one another less than 1.5 times, then all such signals-Sᵢₓⱼ, S_{iyj}, to mention only few, are picked out. The result: a set of elements from various groups ix1, iy1, ix2, iz2, ix3, etc. whose signals exceed the rest of signals in their groups no less than 1.5 times. And if in the set so obtained are present at least two elements from various groups homologous to one subtype, for example, ix1 and ix3 or ix1 and ixy2, a conclusion is drawn on the assayed specimen belonging to the subtype 'x' of a genotype 'i'. Should the elements of different groups in the set so obtained conform to different genotypes, for example, ix1, iy2, iz3 or ix1, iyz3, then the signals of the given elements are compared with each other. If the signal of a element conforming to the subtype 'x' of one group exceeds the signals of the elements of other groups 3 times or more, a conclusion is drawn on the assayed specimen belonging to the subtype 'x'. If a ratio of signals Sᵢₓ₁/S_{iy2} does not exceed 3, a conclusion is drawn on the fact that the subtype is not determined and possible is identity to the subtype 'x' or the subtype 'y'. The same conclusion is drawn if the maximum signal in the group belongs to an element containing an oligonucleotide showing specificity to two subtypes, for example, ixy1, with valid signals absent in other groups of the elements. If the signals of subtype-specific-oligonucleotides-containing groups do not undergo primary filtration with respect to I_{ref}, it is believed that the subtype of a specimen as assayed is not determined.

The evaluation of duration of antiviral therapy and prognosis can be made on the basis of data on genotype/subtype identification. Thus, in case of a genotype 1 being determined that elicits cirrhosis, chronic hepatitis and hepatocarcinoma, duration of pegylated interferon/ribavirin therapy is no less than 24 weeks for subtypes 1a, 1c, 1d, 1e and in case of subtype 1 b being detected that is interferon-resistant - no less than 48 weeks (Weck K. Molecular methods of hepatitis C genotyping. Expert Rev Mol Diagn. 2005 Jul; 5(4): 507-20).

The infection caused by HCV with a genotype 4 provides a clinical picture similar to virus genotype 1 infection (Legrand-Abravanel F, Nicot F, Boulestin A, Sandres-Sauné K, Vinel JP, Alric L, Izopet J. Pegylated interferon and ribavirin therapy for chronic Hepatitis C virus genotype 4 infection. J Med Virol. 2005 Sep; 77(1):66-9). And unlike the genotype 1, the genotype 4 is distinguished for splitting into a considerably greater number of subtypes (Nicot F, Legrand-Abravanel F, Sandres-Saune K, Boulestin A, Dubois M, Alric L, Vinel JP, Pasquier C, Izopet J. 2005. Heterogeneity of Hepatitis C virus genotype 4 strains circulating in south-western France. J Gen Virol Jan; 86(Pt 1): 107-14). The clinical significance of some of them, for example, 4a and 4d has already been established - 4d is resistant to interferon and calls for a prolonged course of treatment (no less than 48 weeks) (Roulot D, Bourcier V, Grando V, Epidemiological characteristics and response to peginterferon plus ribavirin treatment of Hepatitis C virus genotype 4 infection (J Viral Hepat. 2007 Jul; 14(7): 460-7).

The subtypes of HCV genotypes 2 and 3 are responsive to therapy with drugs and lead to chronic disease in significantly lesser amount of cases. Duration of ribavirin/interferon therapy for HCV infected patients with the given genotypes is 6 to 12 weeks.

Apart from prognostic purposes and evaluation of duration of therapy, the definition of a genotype and subtype provides information in terms of etiology of infection. Subtypes 1a, 3a, 4a, 4d are most commonly associated with intravenous drug users, whereas a genotype 2 and a subtype 1 are linked with a blood transfusion transfer route (Simmonds P, Bukh J, Combet C. Consensus proposals for a unified system of nomenclature of Hepatitis C virus genotypes. Hepatology. 2005 Oct; 42(4): 962-73).

Lastly the results obtained by means of the method of the present invention can be made use of for epidemiological genotyping. Distribution of HCV genotypes and subtypes is varied in various geographic regions (Zein NN. Clinical significance of Hepatitis C virus genotypes. Clin Microbiol Rev. 2000 13(2):223-35). Some subtypes are universal, other circulate only within limited geographic zones. Subtype 1b prevails in the south of Europe, China, Japan and in Russia (50-80%). In the USA and countries of South America, in the north of West Europe, subtypes 1a and 1b are dominated, followed by genotypes 2 and 3. Genotype 4 prevails in North Africa and Central Africa, whereas in the south of the continent, a genotype 5 is predominant. Genotype 3 is met almost everywhere whose domain are Australia and South-East Asia. Genotype 6 is likewise widespread in South-East Asia and is predominant in Vietnam, a major type in Thailand, Indonesia.

The invention will now be exemplified to grasp a better idea of a concept of invention, as being claimed and as set forth in the application, but the illustrative examples shouldn't be considered restricting the present invention.

### Example 1. Biochip for identifying HCV genotype and subtype on the basis of NS5B region assay.

1. Oligonucleotides for immobilization on a biochip and primers for amplification were synthesized on an automatic synthesizer-394 DNA/RNA synthesizer (Applied Biosystems, USA) and contained a spacer with a free amino group 3'-Amino-Modifier C7 CPG 500 (Glen Research, USA) for the following immobilization to a gel. Biochips were produced according to the procedure thus far described ((Rubina AY, Pan'kov SV, Dementieva EI et al. Hydrogel drop microchips with immobilized DNA: properties and methods for large-scale production. Anal Biochem 2004; 325: 92-106). The biochips contained hemispherical elements, 100 mcm in dia., through a distance of 300 mcm. Uniformity of the application of elements and their dia. were assessed with the help of the software 'Test-chip' (Biochip-IMB, Russia). The qualitative control of microchips was made by measuring the concentrations of immobilized oligonucleotides. The biochips were stained with a fluorescent dye-ImD-310 (Biochip-IMB), the concentration of immobilized probes was assessed as described above (A.L. Mikheikin, A.V. Choudinov, A.I. Yaroschuk, A.Yu. Roubina, S.V. Pan'kov, A.S. Krylov, A.S. Zasedatelev, A.D. Mirzabekov. A dye showing a low specificity to a DNA nucleotide sequence: use for the evaluation of the number of nucleotides immobilized in biological microchip elements. Molecular biology 2003; 37(6): 1061-70).

### Biochip structure

A biochip comprises 120 immobilized oligonucleotides whose list is presented in Table I, four marker points **(M)** for accurate positioning (image acquisition), performed by a software, and four elements of an empty gel **(O)** necessary for computing a reference (background) value of fluorescence intensity I_{ref}. Arrangement of oligonucleotides immobilized on a microchip is shown in Fig. 3.

In two top rows, oligonucleotides are immobilized with a 'G' index permitting identifying a HCV genotype. A biochip identifies all six HCV genotypes.

Immobilized below are oligonucleotides allowing for identifying subtypes:
- inside a genotype 1: 1a, 1b, 1c, 1d, 1e. For the reliable identification of each and every subtype of the genotype 1 four groups of oligonucleotides have been constructed, each group is conforming to a separate segment within an NS5B region fragment as assayed:
- inside a genotype 2: 2a, 2b, 2c, 2d, 2i, 2j, 2k, 21, 2m. For the reliable identification of each and every subtype of the genotype 2 three groups of oligonucleotides have been constructed, each group is conforming to a separate segment within an NS5B region fragment as assayed;
- inside a genotype 3: 3a, 3b, 3k. For the reliable identification of each and every subtype of the genotype 3 three groups of oligonucleotides have been constructed, each group is conforming to a separate segment within an NS5B region fragment as assayed;
- inside a genotype 4: 4a, 4c, 4d, 4f, 4h, 4i, 4k, 4n, 4o, 4p, 4r, 4t. For reliable identification of each and every subtype of the genotype 4 four groups of oligonucleotides have been constructed, each group is conforming to a separate segment within an NS5B region fragment as assayed;
- inside a genotype 5: 5a. The genotype 5 is a single subtype 5a, with the genotype-identifying probes thus identifying the subtype 5a;
- inside a genotype 6: 6a, 6b, 6d, 6g, 6h, 6k. For the reliable identification of each and every subtype of the genotype 6 two groups of oligonucleotides have been constructed, each group is conforming to a separate segment inside an NS5B region fragment as assayed.

**Table 1. List of oligonucleotides immobilized on biochip**

| SEQ ID NO: | Oligonucleotide* | Genotype | Subtype | Group | Sequence 5'→3'** | Position of oligonucleotide in NS5B region sequence (Acc. No M62321) |
|---|---|---|---|---|---|---|
| 1 | G1-1 | 1 | - | G1 | GCC TGT CGA GCY GC | 904-917 |
| 2 | G1-2 | 1 | - | G1 | GCC TGT CGA GCY GCR | 904-918 |
| 3 | G1-3 | 1 | - | G1 | GCC TGT MGA GCY GCR | 904-918 |
| 4 | G1-4 | 1 | - | G1 | GGC TTT AYR TCG GGG G | 776-791 |
| 5 | G2-1 | 2 | - | G2 | TACAGGCGYTGYCGC | 826-840 |
| 6 | G2-2 | 2 | - | G2 | CTG CGG NTA CAG GCG TTG | 819-836 |
| 7 | G2-3 | 2 | - | G2 | CTG CGG NTA CAG GCG YTG | 819-836 |
| 8 | G3-1 | 3 | - | G3 | YCT TGT CTG CGG AGA Y | 939-954 |
| 9 | G3-2 | 3 | - | G3 | GGC TTT ACT GCG GGG G | 776-791 |
| 10 | G4-1 | 4 | - | G4 | CGA CGA RGA GGT CTA YCA GTG | 705-725 |
| 11 | G4-2 | 4 | - | G4 | CGA GGA RGA GGT MTA YCA GTG | 705-725 |
| 12 | G4-3 | 4 | - | G4 | GTG ACC TRG AGC CCG A | 728-743 |
| 13 | G5-1 | 5 | - | G5 | GTG ACT TRC AGC CCG A | 728-743 |
| 14 | G5-2 | 5 | - | G5 | GCA CGC TCC TGG TGT G | 932-947 |
| 15 | G6 | 6 | - | G6 | TGA CAT GTT GGT CTG CG | 933-949 |
| 16 | 1a11 | 1 | 1a | 1 | CAC TGA GAG CGA CAT CC | 684-700 |
| 17 | 1ce1 | 1 | 1c/1e | 1 | CAC TGA GGC TGA TAT CCG | 684-701 |
| 18 | 1a12 | 1 | 1a | 1 | YAT CCG TAC GGA GGA GG | 696-712 |
| 19 | 1bd12 | 1 | 1b/1d | 1 | YAT CCG TGT TGA GGA GTC | 696-713 |
| 20 | 1a2 | 1 | 1a | 2 | CAT CAA GTC CCT CAC YGA | 756-773 |
| 21 | 1b2 | 1 | 1b | 2 | ATA ARG TCG CTC ACA GAG | 757-774 |
| 22 | 1c2 | 1 | 1c | 2 | CCA TAA GGT CTC TCA CAG A | 755-773 |
| 23 | 1d2 | 1 | 1d | 2 | ATA AAG TCG CTC ACC G | 757-772 |
| 24 | 1e2 | 1 | 1e | 3 | ATC AAG TCY TTG ACT GAA AG | 758-776 |
| 25 | 1a31 | 1 | 1a | 3 | AGG CCC GRG CAG C | 893-905 |
| 26 | 1a32 | 1 | 1a | 3 | AGG CCC ARG CAG C | 893-905 |
| 27 | 1 b3 | 1 | 1b | 3 | GCC WCT GCR GCC TGT | 895-909 |
| 28 | 1bd3 | 1 | 1b/1d | 3 | CCW CTG CGG CCT GT | 896-909 |
| 29 | 1c3 | 1 | 1c | 3 | GCC AGT GCA GCC TGT | 895-909 |
| 30 | 1e3 | 1 | 1e | 3 | CAA GGC CCT AGC AGC | 891-905 |
| 31 | 1d3 | 1 | 1d | 3 | GCC ATR GCR GCC TG | 895-908 |
| 32 | 1a4 | 1 | 1a | 4 | CTA YCG CAG GTG CCG | 825-839 |
| 33 | 1b4 | 1 | 1b | 4 | GTT ATC GCC GGT GC | 824-837 |
| 34 | 1c4 | 1 | 1c | 4 | GCT ATC GGC GAT GC | 824-837 |
| 35 | 1d4 | 1 | 1d | 4 | GCT ACC GTC GGT GC | 824-837 |
| 36 | 1e4 | 1 | 1e | 4 | TAT CGC AGA TGC CGT | 826-840 |
| 37 | 2ad1 | 2 | 2a/2d | 1 | CAG AAH TGA GGA GTC CAT A | 699-717 |
| 38 | 2b11 | 2 | 2b | 1 | ACG GAG AGG GAC ATA AG | 685-701 |
| 39 | 2b12 | 2 | 2b | 1 | CAT AAG AAC AGA AGA ATC CA | 696-715 |
| 40 | 2i1 | 2 | 2i | 1 | TCA CYG AAA GRG ACA TCA G | 683-701 |
| 41 | 2cj1 | 2 | 2c/2j | 1 | YAG AAC CGA GGA GTC C | 699-714 |
| 42 | 2k1 | 2 | 2k | 1 | ACG GAG AGR GAT ATC AGG | 685-702 |
| 43 | 211 | 2 | 21 | 1 | ATA CGG ACA GAA GAA TCC | 697-714 |
| 44 | 2m1 | 2 | 2n | 1 | GGG ACA TYC GAR TCG A | 692-707 |
| 45 | 2a2 | 2 | 2a | 2 | TAC ACT CGC TGA CTG AGA | 758-775 |
| 46 | 2b2 | 2 | 2b | 2 | TAC ACT CGC TCA CTG AGA | 758-775 |
| 47 | 2c2 | 2 | 2c | 2 | ATA CAC TCA CTG ACT GAG AG | 757-776 |
| 48 | 2d2 | 2 | 2d | 2 | CTC ACT GAC TGA GAG GCT | 762-779 |
| 49 | 2kc2 | 2 | 2k/2c | 2 | ACA CTC ACT NAC TGA GAG ACT | 759-779 |
| 50 | 2i2 | 2 | 2i | 2 | **TAC ACT CAC TRA CTG AGA GG** | 758-777 |
| 51 | 2j2 | 2 | 2j | 2 | CAT ACA TTC ACT CAC TGA GA | 756-775 |
| 52 | 212 | 2 | 21 | 2 | ATY AAA TCA CTG ACA GAG AG | 757-776 |
| 53 | 2m2 | 2 | 2m | 2 | ATA CAC TCA YTG ACC GAG A | 757-775 |
| 54 | 2a31 | 2 | 2a | 3 | AAA GCY CTA GCG GC | 891-905 |
| 55 | 2a32 | 2 | 2a | 3 | CYC TAG CGG CTT GYA | 896-910 |
| 56 | 2b31 | 2 | 2b | 3 | AAA GCC CTT GCR GC | 892-905 |
| 57 | 2b32 | 2 | 2b | 3 | AAG CCC TCG CRG C | 892-905 |
| 58 | 2c3 | 2 | 2c | 3 | AAG CCA GRG CGG C | 893-905 |
| 59 | 2d3 | 2 | 2d | 3 | CNR RRG CAG CCT G | 896-908 |
| 60 | 2i3 | 2 | 2i | 3 | GCY CAA GCG GCC T | 895-907 |
| 61 | 2k3 | 2 | 2k | 3 | AGG CCC TGG CGG | 893-904 |
| 62 | 2m3 | 2 | 2m | 3 | AAG CCC AAG CAG CC | 893-916 |
| 63 | 3a1 | 3 | 3a | 1 | ACA TCA GGG TGG AAG AG | 695-711 |
| 64 | 3b1 | 3 | 3b | 1 | CAT CAG GAC GGA GGA G | 696-711 |
| 65 | 3a3 | 3 | 3a | 3 | AAG GCC ACR GCG G | 892-904 |
| 66 | 3b3 | 3 | 3b | 3 | AAG GCC ACT GCV GC | 894-905 |
| 67 | 3k3 | 3 | 3k | 3 | AAG CAA AGG CAG CC | 893-906 |
| 68 | 3a2 | 3 | 3a | 2 | ATC TCC TCC CTC ACG G | 757-772 |
| 69 | 3b2 | 3 | 3b | 2 | ATC AGC GCT CTC ACR G | 757-772 |
| 70 | 3k2 | 3 | 3k | 2 | TGA TAA CTT CAC TCA CGG | 755-772 |
| 71 | 4ac1 | 4 | 4a/4c | 1 | AAC CGA AAA GGA CAT CA | 684-700 |
| 72 | 4df1 | 4 | 4d/4f | 1 | TRA CYG AAA GAG ACA TCA | 683-700 |
| 73 | 4hk1 | 4 | 4h/4k | 1 | TGA CTG AAA GGG ACA TCA | 683-700 |
| 74 | 4i1 | 4 | 4i | 1 | CGT GAC GGA GAG AGA CA | 681-697 |
| 75 | 4n1 | 4 | 4n | 1 | ACT GTG ACT GAG AAA GAC A | 679-697 |
| 76 | 4p1 | 4 | 4p | 1 | CCG TGA CTG AGA AGG AC | 680-697 |
| 77 | 4r1 | 4 | 4r | 1 | GTC ACC GAA ARR GAC AT | 682-692 |
| 78 | 4a21 | 4 | 4a | 2 | GTT ATT GCY GCC CTC A | 754-769 |
| 79 | 4dp2 | 4 | 4d/4p | 2 | GGT GAT ATC CGC CCT | 753-767 |
| 80 | 4a22 | 4 | 4a | 2 | GCA AAG TCA TCA CCG CC | 749-765 |
| 81 | 4c2 | 4 | 4c | 2 | ACY GCC CTA ACA GAG AG | 760-776 |
| 82 | 4f2 | 4 | 4f | 2 | AGG TRA TAT CCG CCC T | 752-767 |
| 83 | 4i2 | 4 | 4i | 2 | AGG TCA TCA AMG CCC | 752-766 |
| 84 | 4k2 | 4 | 4k | 2 | ARA CCR ATA TCC GCC CT | 751-767 |
| 85 | 4n2 | 4 | 4n | 2 | GYY ATA ACC GCC CTC A | 754-769 |
| 86 | 4o2 | 4 | 4o | 2 | CGC CCT TAC RGA GAG | 762-776 |
| 87 | 4r2 | 4 | 4r | 2 | AAG GCC ATA ACC GC | 751-764 |
| 88 | 4t2 | 4 | 4t | 2 | GGT RAT AYC AGC CCT CA | 753-769 |
| 89 | 4a3 | 4 | 4a | 3 | CAA AGC CAC AGC CGC | 891-905 |
| 90 | 4c3 | 4 | 4c | 3 | AAA GCC TMA GCC GC | 892-905 |
| 91 | 4d3 | 4 | 4d | 3 | TAA GGC CAG CGC AGC | 891-905 |
| 92 | 4f3 | 4 | 4f | 3 | YAA GGC YAC MGC GGC | 891-905 |
| 93 | 4h3 | 4 | 4h | 3 | ARG CCA CRG CAR CCA | 893-907 |
| 94 | 4k3 | 4 | 4k | 3 | TTA AGG CYG YCG CAG | 890-904 |
| 95 | 4on3 | 4 | 4o/4n | 3 | AAG ACC ACR GCC GCC | 892-907 |
| 96 | 4i3 | 4 | 4i | 3 | CCT CAA RGC CAC AGC | 891-906 |
| 97 | 4p3 | 4 | 4p | 3 | YAA GGC AAC AGG AGC | 891-906 |
| 98 | 4r3 | 4 | 4r | 3 | AAA ACC ACG GCR GCC A | 892-907 |
| 99 | 4a4 | 4 | 4a | 4 | TGT GGG TAT CGG AGA TG | 820-836 |
| 100 | 4c4 | 4 | 4c | 4 | CGG GTA TCG CAG ATG | 822-836 |
| 101 | 4d4 | 4 | 4d | 4 | CGG RAC TCG ACG GTG | 822-836 |
| 102 | 4f4 | 4 | 4f | 4 | YGG GTA CCG TAG ATG C | 822-837 |
| 103 | 4h4 | 4 | 4h | 4 | CGG GHT TCG GAG GT | 822-835 |
| 104 | 4i4 | 4 | 4i | 4 | GTG GCA TCC GTA GAT G | 821-836 |
| 105 | 4k4 | 4 | 4k | 4 | GCG GGT ATC GVA GGT G | 821-836 |
| 106 | 4o4 | 4 | 4o | 4 | GCC AGC GGA GAT GC | 824-837 |
| 107 | 4pt4 | 4 | 4p/4t | 4 | CGG TGT BCG YAG GTG C | 822-837 |
| 108 | 4r4 | 4 | 4r | 4 | CGG TTA TCG GAG ATG C | 822-837 |
| 109 | 5a | 5 | 5a | 1 | GYR ATA CGG TCA CTC AC | 754-770 |
| 110 | 6a1 | 6 | 6a | 1 | CGG ACT GAG AAC GAC AT | 700-716 |
| 111 | 6b1 | 6 | 6b | 1 | CGA ACT GAA GAG GAC ATC | 700-717 |
| 112 | 6d1 | 6 | 6d | 1 | CGG ACT GAG GAG GAC AT | 700-716 |
| 113 | 6g1 | 6 | 6g | 1 | CGG ACA GAG GAG TCY AT | 700-716 |
| 114 | 6h1 | 6 | 6h | 1 | CGC ACA GAA CAA GAC AT | 700-716 |
| 115 | 6k1 | 6 | 6k | 1 | ACT GAG CGG GAT GTC T | 703-718 |
| 116 | 6a3 | 6 | 6a | 3 | GCA CAG GCC GCC T | 895-907 |
| 117 | 6b3 | 6 | 6b | 3 | GCA CAG GCG GCG T | 895-906 |
| 118 | 6d3 | 6 | 6d | 3 | AGG CGC AAG CAG C | 893-905 |
| 119 | 6g3 | 6 | 6g | 3 | AGG CCA YGG CGG | 893-904 |
| 120 | 6h3 | 6 | 6h | 3 | GCA ACC GCC GCT | 895-906 |

| | | | | | | |
|---|---|---|---|---|---|---|
| • * Name of oligonucleotides in accordance with positions thereof in Fig 3 • ** Meaning of the one-letter codes for nucleotides in degenerate oligonucleotide sequences: R-A,G Y-C,T M-A,C K-G,T S-C,G W-A,T H-A,C,T B-C,G,T V-A,C,G D-A,G,T **N-A,C,G,T** | | | | | | |

### Example 2. Reverse transcription combined with polymerase chain reaction (RT-PCR) of NS5B region fragment; production of single-stranded fluorescent labeled fragment by asymmetric PCR

First step: reverse transcription combined with the PCR (RT-PCR) to obtain a 418 b.p. NS5B region fragment.

A 10 mcl of isolated viral RNA was added to 40 mcl RT-PCR mix (final volume of 50 mcl).

Mix for RT-PCR included:
- 1X RT-PCR buffer: 70 mM Tris-1-HCl, pH 8.3, 16.6 mM (NH₄)₂SO₄, 7.5 mM MgCl₂;
- dATP, dCTP, dGTP, dUTP at a concentration 200 µmol/L each (Sileks, Russia);
- primers (sequences are presented in Table 2) Pr3_f/Pr2_r at a concentration of 200 nM each;
- 10 units of thermostable ST-polymerase (Sileks, Russia);
- 1 unit of uracil-DNA-glycosylase (Sileks, Russia);
- 10 units of RNAse inhibitor (Fermentas, Lithuania).

Amplification was carried out on thermocycler PTC-200 Dyad (MJ Research, USA): reverse transcription at 50°C-30 min, followed by 50 cycles of PCR: 95°C-30 s, 63°C-30 s, 72°C- 30 s; final elongation at 72°C - 10 min.

A 1 mcl reaction mix obtained at first step of amplification was used as template for second step.

A second PCR step was carried out in a semi-nested variant with P3_f/Pr5_r primers flanking a 382 b.p. NS5B region. Primer sequences are given in Table 2.

Mix for RT-PCR included (25 mcl):
- 1X PCR-buffer: 10 mM KCl, 10 mM Tris-HCl (pH 8.3) (Sileks, Russia);
- 1.5 mM MgCl₂;
- dATP, dCTP, dGTP, dUTP at a concentration 200 µmol/L each (Sileks, Russia);
- fluorescent labeled dUTP at a concentration 10 µmol/L ("Biochip-IMB", Russia);
- P3_f/Pr5_r primers at a concentration of 20 nm/100 nM, respectively;
- 10 units of thermostable Taq DNA-polymerase (Sileks, Russia).

Amplification was performed on thermocycler PTC-200 Dyad (MJ Research, USA): 95°C-2 min, then 36 cycles: 95°C - 20s, 60°C-20 s, 72°C - 30 s, final elongation: 72°C - 5 min. 12 mcl of the obtained product were used in hybridization on a biochip.

**Table 2. List of primers for the amplification of an NS5B region fragment.**

| SEQ ID NO: | Title | Sequence 5'→ 3'* | Primer position in NS5B fragment sequence (Acc. No M62321) |
|---|---|---|---|
| 121 | Pr3_f | TATGAYACCCGCTGYTTTGACTC | 655-677 |
| 122 | Pr2_r | GGCGGAATTCCTGGTCATAGCCTCCG TGAA | 1015-1044 |
| 123 | Pr5_r | GCTAGTCATAGCCTCCGT | 1018-1035 |

| | | | |
|---|---|---|---|
| * one-letter code for nucleotide in degenerate primer sequence: Y=C,T | | | |

### Example 3. Hybridization of amplified labeled product on biochip

A 12 mcl reaction mix obtained after a second step of PCR and predominantly comprising DNA single-stranded fluorescent labeled fragments conforming to an NS5B region fragment, as assayed, was added with the concentrated solution of a hybridization buffer such that the final concentration of guanidine thiocyanate was 1 M, HEPES - 50 mM, pH 7.5, EDTA - 5 mM. The reaction chamber of the biochip was filled with the 32 mcl of resulted hybridization mixture and sealed. Hybridization was performed at 37°C for 12-18 hours. On completion of hybridization, the biochip was washed thrice with distilled water at 37°C for 30 s and dried.

### Example 4. Registration and interpretation of hybridization results

The registration of fluorescence pattern of biochip was performed using universal fluorescence analyzer (Biochip-IMB, Ltd, Russia) equipped with specialized software 'ImageWare^{®}' (Biochip-IMB, Ltd, Russia).

The interpretation of results was performed by the aforesaid algorithm of a software module for the analysis of the fluorescent images of biochips 'Imageware^{®}'.

### Example 5. Analysis of NS5B region of HCV sample belonging to subtype 1a using hybridization on biochip

An HCV viral RNA was isolated from patient's blood specimen using Qiamp Viral RNA mini kit (Qiagen, Germany) under the protocol of manufacturer. The isolated RNA was used in RT - PCR as described (Example 2). The presence of an amplified NS5B 418 b.p. long fragment was tested by electrophoresis in agarose gel whereupon a RT-PCR product was divided into two portions of which one was treated and assayed according to the methods as described in Examples 2-4 (a second PCR step followed by hybridization on a biochip, washing, registration and interpretation of the fluorescent pattern of the biochip). The second portion of a first step product was used upon additional purification in sequencing reaction, followed by analysis on an automatic sequencer, correcting a chromatogram and obtaining the sequence of NS5B region fragment, constructing a multiple alignment and a phylogenetic tree on whose basis a genotype and a subtype were determined.

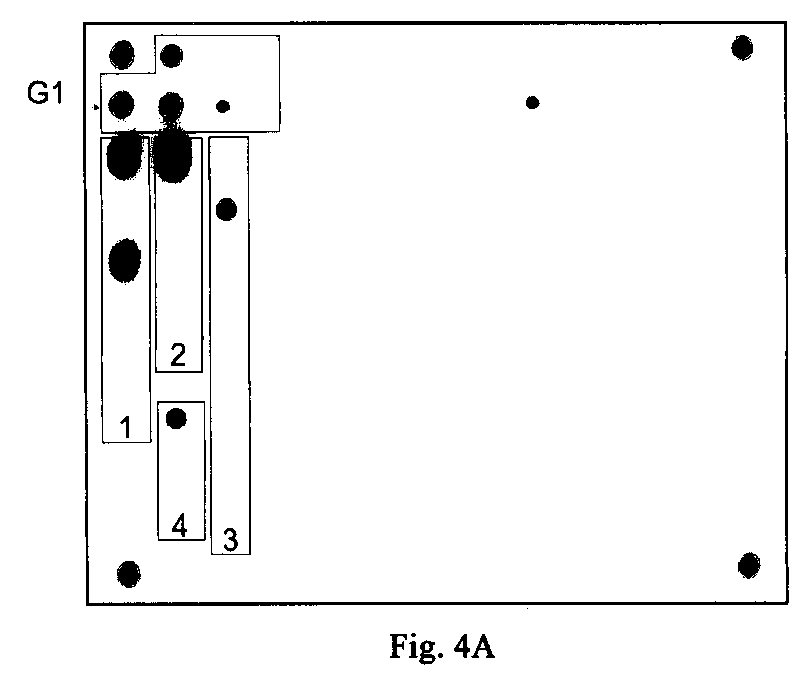

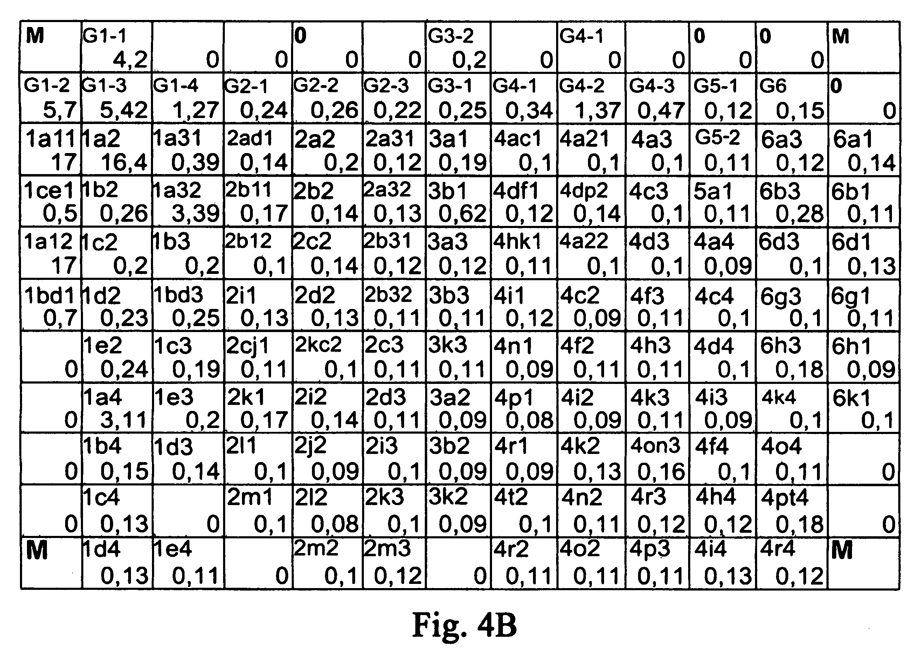

Fig. 4A shows a biochip hybridization pattern. Fig. 4B demonstrates the distribution of the normalized fluorescence signals of biochip elements.

In accordance with the algorithm of results interpretation, as suggested, analysis starts off with computing a mean signal (I_{ref}) in empty elements, in which particular case the I_{ref} is 0.62. In accordance with this value and the threshold value of 1.5, the signals are filtered into genotype and subtype-specific elements, with the result that the signals in group G1 containing genotype 1-specific probes exceed the I_{ref} 1.5 times or more. A similar situation is evolved around the signal in a element G4-2 (1.37). The signals in other groups containing genotype-specific probes were close to background ones. Furthermore, the maximum signal is detected from group G1, G1-2 (5.7). The signal in the given element exceeds a signal G4-2 more than 1.5 times. So the sequence of the analyzed HCV sample, as assayed, relates to a genotype 1.

An analysis in the groups of elements comprising subtype-specific probes belonging to genotype 1 goes to show: In group I, the maximum (exceeding a 1.5 threshold value with respect to the remaining elements) signals have 1a11 (17.0) and 1a12 (17.0). In group 2 - 1a2 (16.4). In group 3 - 1a32 (3.4). In group 4 - 1a4 (3.1). Thus, in all the groups, a maximum signal is characteristic of the elements comprising the probes specific for subtype 1a. This means, that the analyzed HCV specimen is related to the subtype 1a.

A sequencing method with subsequent phylogenetic analysis showed that the sequence being assayed falls within a cluster of subtype 1a sequences.

Thus, it has been established that an HCV RNA specimen, as assayed, has a genotype 1 and a subtype 1a, which coincides with sequencing results in full.

### Example 6. Analysis of NS5B region of HCV sample belonging to subtype 1b using hybridization on biochip

An HCV viral RNA was isolated from patient's blood specimen using Qiamp Viral RNA mini kit (Qiagen, Germany) under the protocol of manufacturer. The isolated RNA was used in RT - PCR as described (Example 2). The presence of an amplified NS5B 418 b.p. long fragment was tested by electrophoresis in agarose gel whereupon a RT-PCR product was divided into two portions of which one was treated and assayed according to the methods as described in Examples 2-4 (a second PCR step followed by hybridization on a biochip, washing, registration and interpretation of the fluorescent pattern of the biochip). The second portion of a first step product was used upon additional purification in sequencing reaction, followed by analysis on an automatic sequencer, correcting a chromatogram and obtaining the sequence of NS5B region fragment, constructing a multiple alignment and a phylogenetic tree on whose basis a genotype and a subtype were determined.

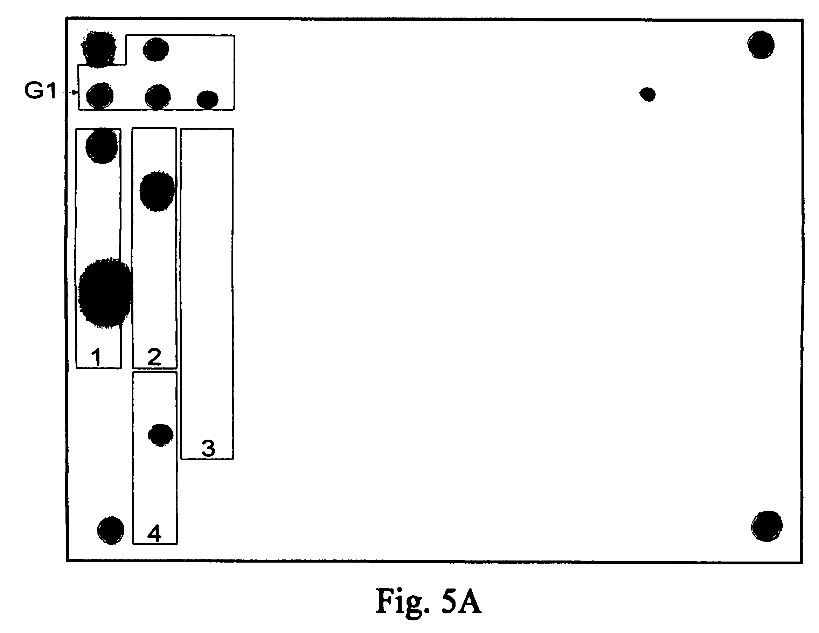

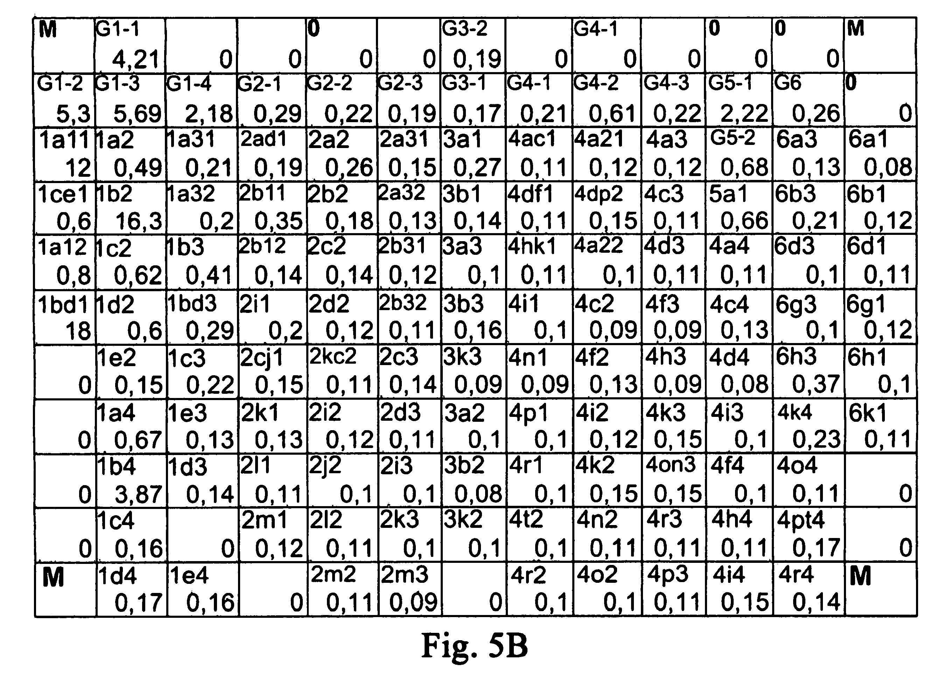

Fig. 5A shows a biochip hybridization pattern. Fig. 5B demonstrates the distribution of the normalized fluorescence signals of biochip elements.

In accordance with the algorithm of results interpretation, as suggested, analysis starts off with computing a mean signal (I_{ref}) in empty elements, in which particular case the I_{ref} is 0.67. In accordance with this value and the threshold value of 1.5, the signals are filtered in genotype-and subtype - specific elements. The result is that the signals in G 1 group containing genotype 1 - specific probes exceed the I_{ref} 1.5 times or more (the maximum signal is characteristic of a G1-3 element (5.69)). The signals in other groups containing the genotype-specific probes were close to background ones. So the sequence of the analyzed HCV sample, as assayed, relates to a genotype 1.

Analysis in the groups of elements containing subtype-specific probes of genotype 1 subtypes reveals the following: in group 1, the maximum (i.e. exceeding a threshold value 1.5 times vs other elements) signal has 1bd1 (18.0). In group 2 - 1b2 (16.3). In group 4 - 1b4 (3.9). In group 1 a perfect duplex with a DNA, as assayed, forms also an oligonucleotide whose sequence is universal for subtypes 1b and 1d. However, the maximum signal is registered also in the elements containing probes specific only for the subtype 1b - 1b2 and 1b4. Consequently, the assayed specimen relates to the subtype 1b.

A sequencing method with subsequent phylogenetic analysis showed that the sequence being assayed falls within a cluster of subtype 1b sequences.

Thus, it has been established that an HCV RNA specimen, as assayed, has a genotype 1 and a subtype 1 b which fully coincides with sequencing results.

### Example 7. Analysis of NS5B region of HCV sample belonging to subtype 1e using hybridization on biochip

An HCV viral RNA was isolated from patient's blood specimen using Qiamp Viral RNA mini kit (Qiagen, Germany) under the protocol of manufacturer. The isolated RNA was used in RT - PCR as described (Example 2). The presence of an amplified NS5B 418 b.p. long fragment was tested by electrophoresis in agarose gel whereupon a RT-PCR product was divided into two portions of which one was treated and assayed according to the methods as described in Examples 2-4 (a second PCR step followed by hybridization on a biochip, washing, registration and interpretation of the fluorescent pattern of the biochip). The second portion of a first step product was used upon additional purification in sequencing reaction, followed by analysis on an automatic sequencer, correcting a chromatogram and obtaining the sequence of NS5B region fragment, constructing a multiple alignment and a phylogenetic tree on whose basis a genotype and a subtype were determined.

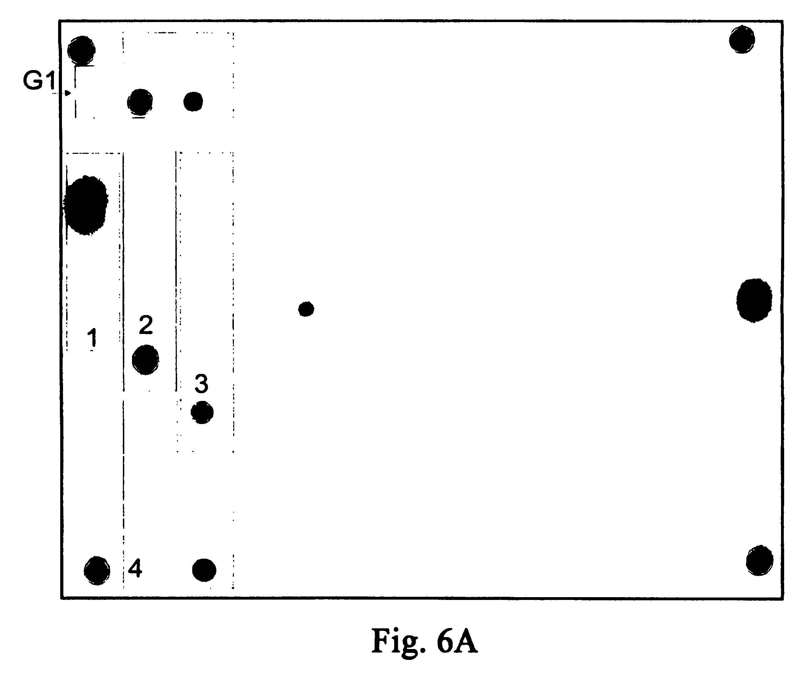

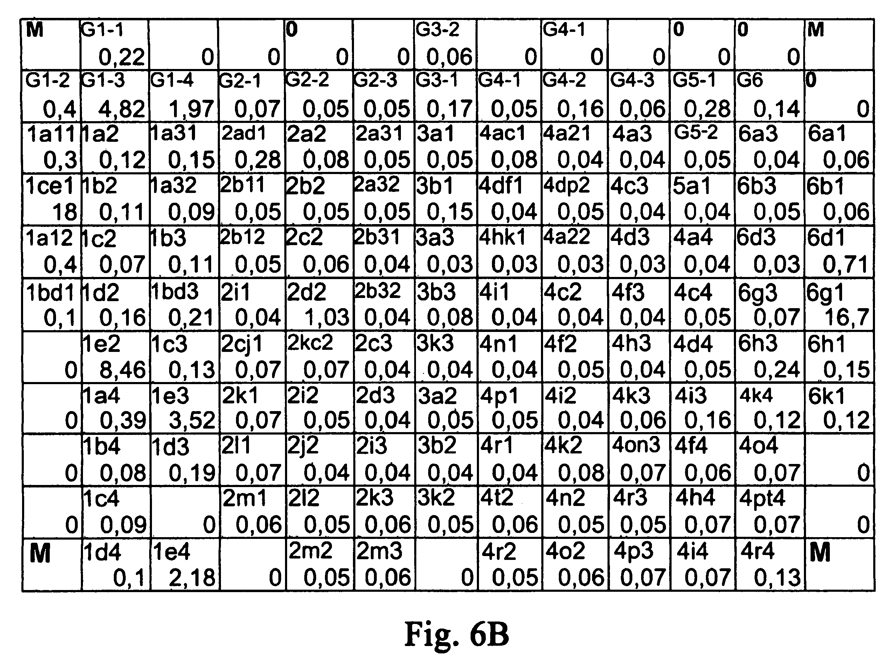

Fig. 6A shows a biochip hybridization pattern. Fig. 6B demonstrates the distribution of the normalized fluorescence signals of biochip elements.

In accordance with the algorithm of results interpretation, as proposed, analysis begins with the computation of a mean signal (I_{ref}) in empty elements. Here the value of I_{ref} is 0.39. According to this value and the threshold value of 1.5, the signals are filtered in genotype-and subtype-specific elements. The result: the signals in group G1 comprising probes showing specificity to a genotype 1 exceed the I_{ref} 1.5 times or more. The signals in other groups of elements containing the genotype-specific probes were close to background ones. The maximum signal in the group G1 is characteristic of a G1-3 element (4.82). So the sequence of the analyzed HCV sample relates to a genotype 1.

Analysis in the groups of elements containing probes specific for genotype 1 subtypes goes to show: in group I, the maximum (exceeding the threshold value of 1.5 with respect to other elements) signal has 1cel (18.0). In group 2 - 1e2 (8.46). In group 3 - 1e3 (3.52). In group 4 - 1e4 (2.18). The perfect duplex with target hybridized NS5B fragment in group 1 was formed by the oligonucleotide with the sequence matching the subtypes 1c and le. However, the maximum signal is also registered in elements containing probes specific only for subtypes 1e -1e2, 1e3 and 1e4. If follows that the specimen, as assayed, is related to the subtype 1e.

A sequencing method with subsequent phylogenetic analysis showed that the sequence being assayed falls within a cluster of subtype le sequences.

Thus, it has been established that an HCV RNA specimen as assayed has a genotype I and a subtype 1e, which fully coincides with sequencing results.

### Example 8. Analysis of NS5B region of HCV sample belonging to subtype 2a using hybridization on biochip

An HCV viral RNA was isolated from patient's blood specimen using Qiamp Viral RNA mini kit (Qiagen, Germany) under the protocol of manufacturer. The isolated RNA was used in RT - PCR as described (Example 2). The presence of an amplified NS5B 418 b.p. long fragment was tested by electrophoresis in agarose gel whereupon a RT-PCR product was divided into two portions of which one was treated and assayed according to the methods as described in Examples 2-4 (a second PCR step followed by hybridization on a biochip, washing, registration and interpretation of the fluorescent pattern of the biochip). The second portion of a first step product was used upon additional purification in sequencing reaction, followed by analysis on an automatic sequencer, correcting a chromatogram and obtaining the sequence of NS5B region fragment, constructing a multiple alignment and a phylogenetic tree on whose basis a genotype and a subtype were determined.

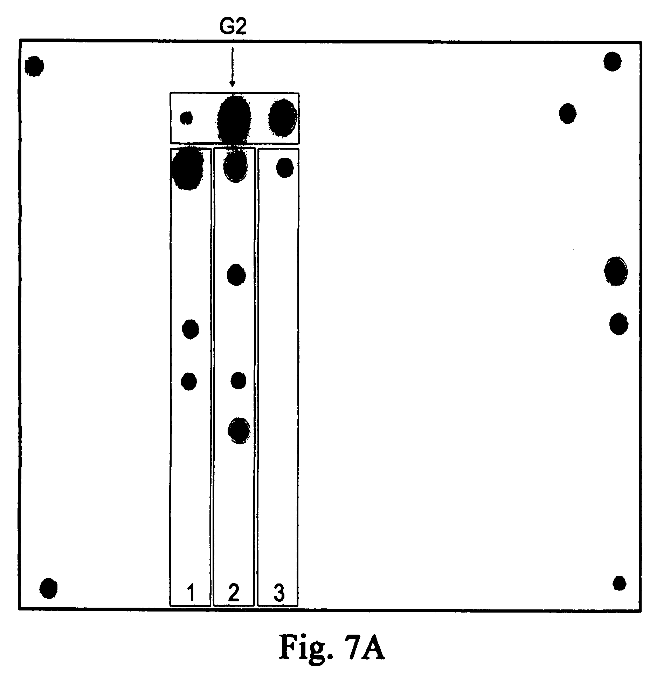

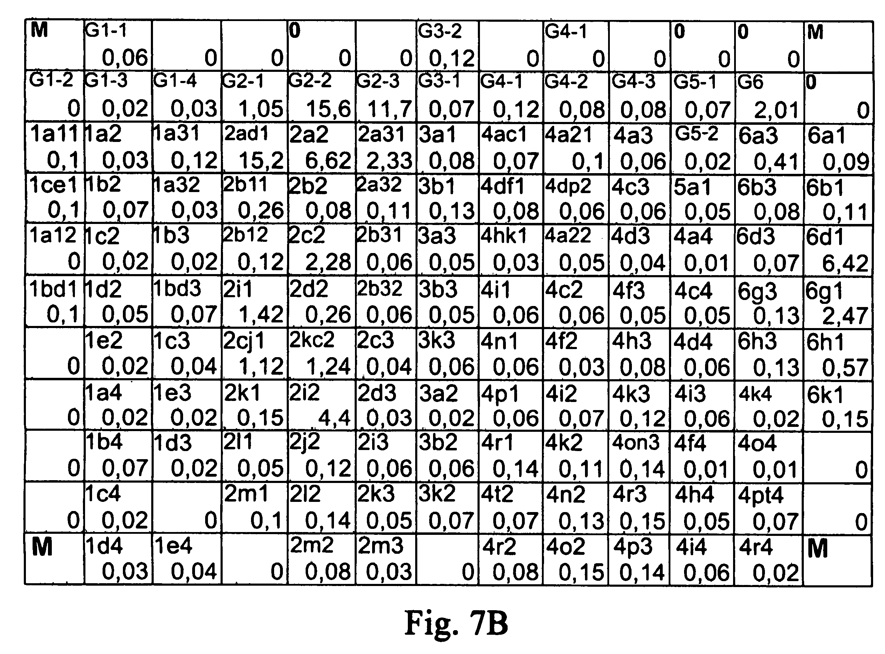

Fig. 7A shows a biochip hybridization pattern. Fig. 7B demonstrates the distribution of the normalized fluorescence signals of biochip elements.

In accordance with the algorithm of results interpretation, as proposed, analysis begins with the computation of a mean signal (I_{ref}) in empty elements. In this case, the value of I_{ref} is 0.25. According to this value and the 1.5 threshold value, filtration of the signals is carried out in genotype- and subtype-specific elements. As a result, the signals in group 2 elements comprising probes showing specificity to a genotype 2 exceed the I_{ref} 1.5 times or more. The signal in a G6 element (2.01) is likewise valid relative to the I_{ref} The maximum signal in the group 2 is characteristic of an element G2-2 (15.6) exceeding the signal in a G6 element more than 1.5 times. It follows that the sequence of the analyzed HCV sample relates to a genotype 2.

Assaying in the groups of elements comprising the subtype-specific probes of genotype 2 has revealed: in group 1 the maximum (i.e. exceeding a 1.5 threshold value in relation to other elements) signal has 2ad1 (15.2). In group 2-2a2 (6.62). In group 3 - 2a31 (2.33). In all three groups, the maximum signal is characteristic of the elements containing probes specific for a subtype 2a. So, the specimen as assayed refers to the subtype 2a.

A sequencing method with subsequent phylogenetic analysis showed that the sequence being assayed falls within a cluster of subtype 2a sequences.

It is hence only logical to deduce that an HCV RNA specimen as assayed has a genotype 2 and a subtype 2a, which fully coincides with sequencing results.

### Example 9. Analysis of NS5B region of HCV sample belonging to subtype 2i using hybridization on biochip

An HCV viral RNA was isolated from patient's blood specimen using Qiamp Viral RNA mini kit (Qiagen, Germany) under the protocol of manufacturer. The isolated RNA was used in RT - PCR as described (Example 2). The presence of an amplified NS5B 418 b.p. long fragment was tested by electrophoresis in agarose gel whereupon a RT-PCR product was divided into two portions of which one was treated and assayed according to the methods as described in Examples 2-4 (a second PCR step followed by hybridization on a biochip, washing, registration and interpretation of the fluorescent pattern of the biochip). The second portion of a first step product was used upon additional purification in sequencing reaction, followed by analysis on an automatic sequencer, correcting a chromatogram and obtaining the sequence of NS5B region fragment, constructing a multiple alignment and a phylogenetic tree on whose basis a genotype and a subtype were determined.

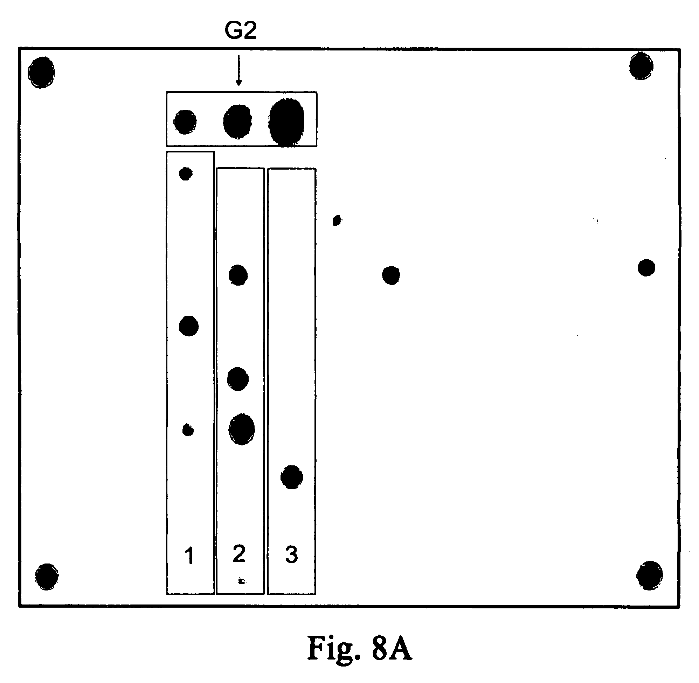

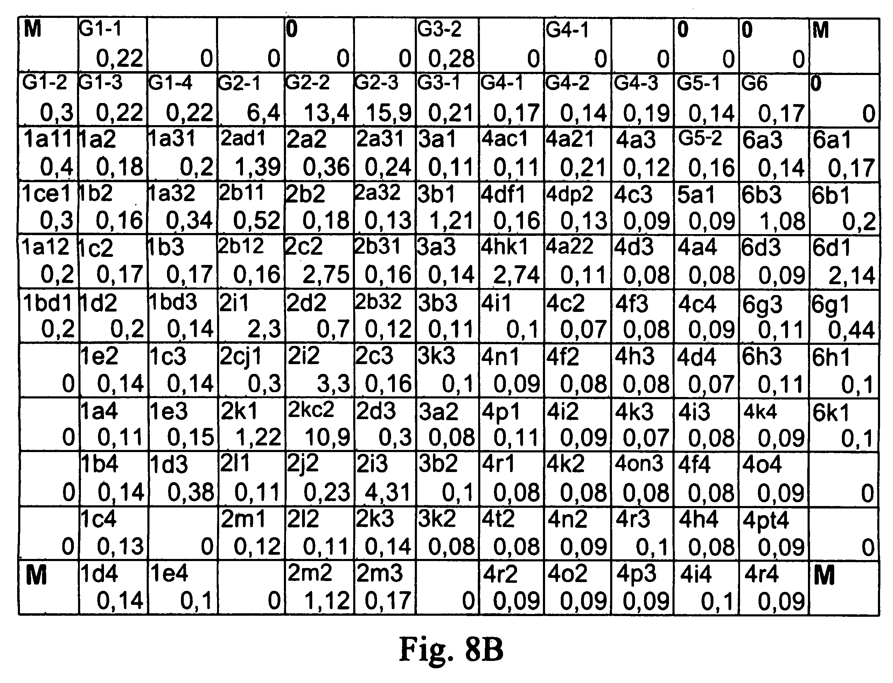

Fig. 8A shows a biochip hybridization pattern. Fig. 8B demonstrates the distribution of the normalized fluorescence signals of biochip elements.

In accordance with the algorithm of results interpretation, as proposed, analysis begins with the computation of a mean signal (I_{ref}) in empty elements. In this case, the value of I_{ref} is 0.44. In accordance with this value and a 1.5 threshold value, the signals are filtered in genotype- and subtype-specific elements with the result that only the signals in elements of group G2 with genotype 2-specific probes exceed the I_{ref} 1.5 times or more. The maximum signal in the group G2 is characteristic of an element C2-3 (15.9). It follows that the sequence of the analyzed HCV sample relates to a genotype 2.

Assaying in the groups of elements comprising the subtype-specific probes of genotype 2 has revealed: in group I, the maximum (i.e. exceeding a 1.5 threshold value relative to other elements) signal has 2i1 (2.3). In group 2 - 2kc2 (10.9). In group 3 - 2i3 (4.31). In group 2, the perfect duplexes are provided with an assayed DNA with an oligonucleotide whose sequence is universal for subtypes 2c and 2k. However, in two other groups, the maximum signal belongs to the elements containing unique oligonucleotides showing a specificity to a subtype 2i. In accordance with the presently claimed algorithm of interpretation, the specimen as assayed refers to the subtype 2i.

A sequencing method with subsequent phylogenetic analysis showed that the sequence being assayed falls within a cluster of subtype 2i sequences.

Thus, it has been ascertained that an HCV RNA specimen as assayed has a genotype 2 and a subtype 2i, which fully coincides with sequencing results.

### Example 10. Analysis of NS5B region of HCV sample belonging to subtype 3a using hybridization on biochip

An HCV viral RNA was isolated from patient's blood specimen using Qiamp Viral RNA mini kit (Qiagen, Germany) under the protocol of manufacturer. The isolated RNA was used in RT - PCR as described (Example 2). The presence of an amplified NS5B 418 b.p. long fragment was tested by electrophoresis in agarose gel whereupon a RT-PCR product was divided into two portions of which one was treated and assayed according to the methods as described in Examples 2-4 (a second PCR step followed by hybridization on a biochip, washing, registration and interpretation of the fluorescent pattern of the biochip). The second portion of a first step product was used upon additional purification in sequencing reaction, followed by analysis on an automatic sequencer, correcting a chromatogram and obtaining the sequence of NS5B region fragment, constructing a multiple alignment and a phylogenetic tree on whose basis a genotype and a subtype were determined.

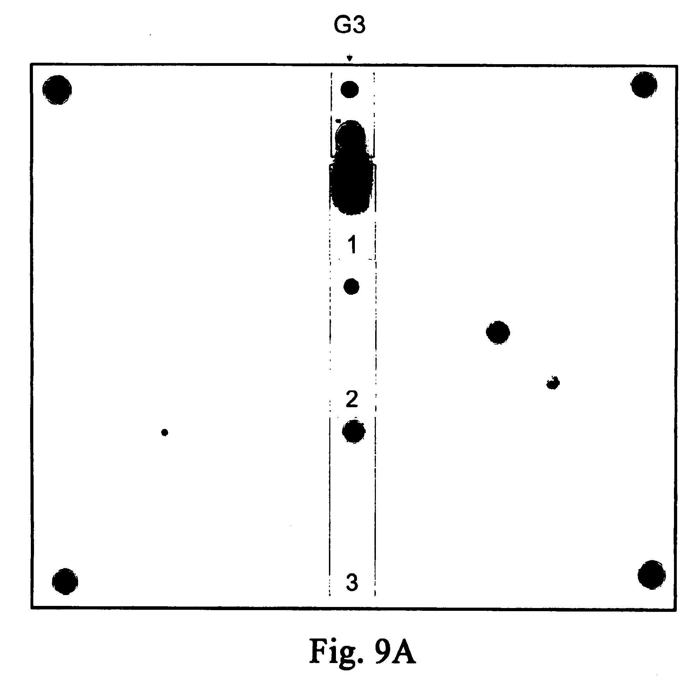

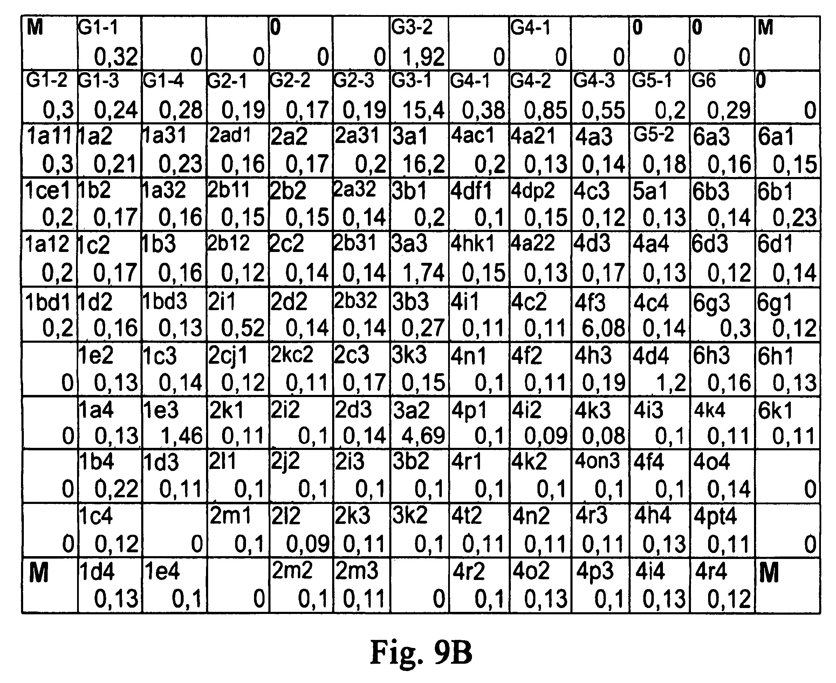

Fig. 9A shows a biochip hybridization pattern. Fig. 9B demonstrates the distribution of the normalized fluorescence signals of biochip elements.

In accordance with the algorithm of results interpretation, as proposed, analysis begins with the computation of a mean signal (I_{ref}) in empty elements. In this case, the value of I_{ref} is 0.52. According to this value and a 1.5 threshold value, the signals are filtered in genotype- and subtype-specific elements. As a result, the signals in group G3 containing probes specific for a genotype 3 exceed the I_{ref} 1.5 times or more. The signal in a G4-2 element (0.85) likewise exceeds the I_{ref} 1.5 times. The signals in other groups of elements containing genotype-specific probes are close to background ones. The maximum signal in the group G3 is characteristic of a G3-1 element (15.4) whose signal exceeds that of G4-2 more than 1.5 times. It follows that the sequence of the analyzed HCV sample relates to a genotype 3.

Assaying in the groups of elements containing subtype-specific probes of genotype 3 reveals the following: in group I the maximum (i.e. exceeding a 1.5 threshold value relative to other elements) signal is characteristic of a 3a1 element (16.2). In group 2 - 3a2 (4.69). In group 3 - 3a3 (1.74). And, as so, in all the groups, the maximum signal is featured by probe-containing elements showing a specificity to a subtype 3a. Consequently the specimen as assayed is related to the subtype 3a.

A sequencing method with subsequent phylogenetic analysis showed that the sequence being assayed falls within a cluster of subtype 3a sequences.

Thus, it has been established that an HCV RNA specimen as assayed has a genotype 3 and a subtype 3a, which is in full coincidence with sequencing results.

### Example 11. Analysis of NS5B region of HCV sample belonging to subtype 4a using hybridization on biochip

An HCV viral RNA was isolated from patient's blood specimen using Qiamp Viral RNA mini kit (Qiagen, Germany) under the protocol of manufacturer. The isolated RNA was used in RT - PCR as described (Example 2). The presence of an amplified NS5B 418 b.p. long fragment was tested by electrophoresis in agarose gel whereupon a RT-PCR product was divided into two portions of which one was treated and assayed according to the methods as described in Examples 2-4 (a second PCR step followed by hybridization on a biochip, washing, registration and interpretation of the fluorescent pattern of the biochip). The second portion of a first step product was used upon additional purification in sequencing reaction, followed by analysis on an automatic sequencer, correcting a chromatogram and obtaining the sequence of NS5B region fragment, constructing a multiple alignment and a phylogenetic tree on whose basis a genotype and a subtype were determined.

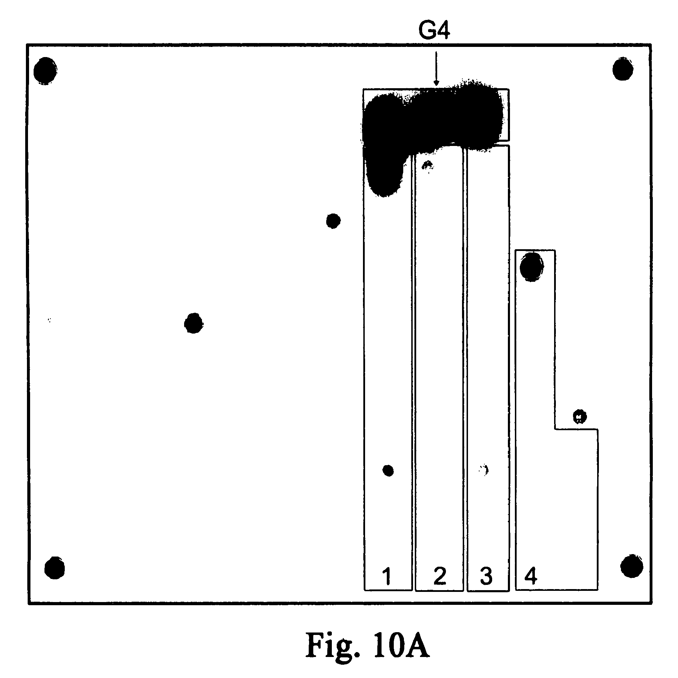

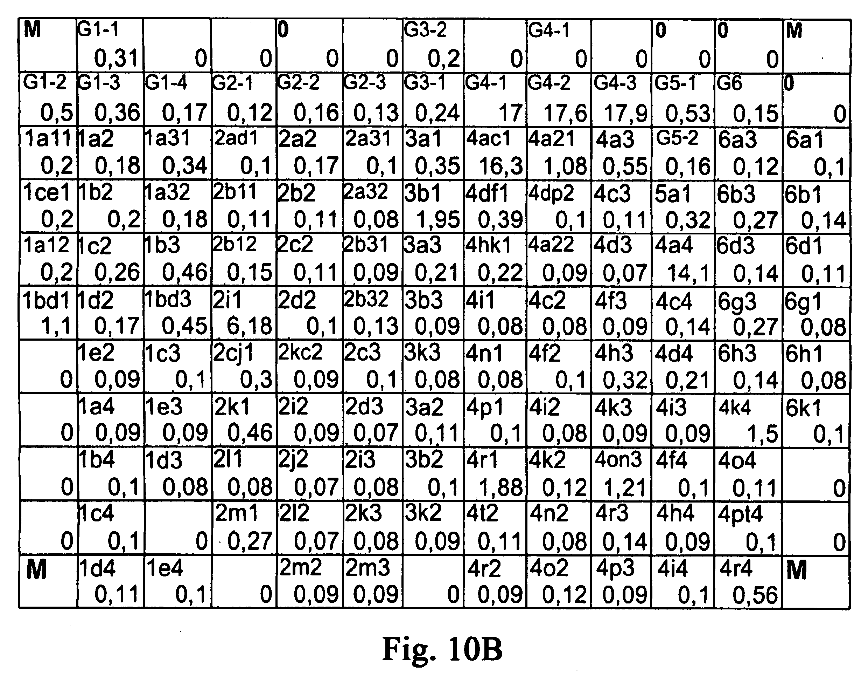

Fig. 10A shows a biochip hybridization pattern. Fig. 10B demonstrates the distribution of the normalized fluorescence signals of biochip elements.

In accordance with the algorithm of results interpretation, as proposed, analysis begins with the computation of a mean signal (I_{ref}) in empty elements. In this case, the value of I_{ref} is 0.55. In accordance with this value and a 1.5 threshold value, the signals are filtered in genotype-and subtype-specific elements. As a result, only the signals in G4 group elements containing probes specific for a genotype 4 exceed the I_{ref} 1.5 times or more. The signals in the remaining elements containing genotype-specific oligonucleotides were close to background ones. The maximum signal in the G4 group is registered in a G4-3 element (17.9). It follows that the RNA sequence of a specimen as assayed is related to the genotype 4.

Assaying in groups containing subtype-specific probes of genotype 4 reveals the following points: in three groups of probes specific for a genotype 4, the maximum signals belong to the elements containing probes for detecting a subtype 4a: 4ac1 (16.3), 4a21 (1.08), 4a4 (14.1). In group 3, the maximum signal belongs to a 4on3 element (1.21); however, in accordance with the algorithm, as shown and described, the specimen as assayed refers to the subtype 4a.

A sequencing method with subsequent phylogenetic analysis showed that the sequence being assayed falls within a cluster of subtype 4a sequences.

With that so, it has been established that an HCV RNA specimen as assayed has a genotype 4 and a subtype 4a, which is in full coincidence with sequencing results.

### Example 12. Analysis of NS5B region of HCV sample belonging to subtype 4d using hybridization on biochip

An HCV viral RNA was isolated from patient's blood specimen using Qiamp Viral RNA mini kit (Qiagen, Germany) under the protocol of manufacturer. The isolated RNA was used in RT - PCR as described (Example 2). The presence of an amplified NS5B 418 b.p. long fragment was tested by electrophoresis in agarose gel whereupon a RT-PCR product was divided into two portions of which one was treated and assayed according to the methods as described in Examples 2-4 (a second PCR step followed by hybridization on a biochip, washing, registration and interpretation of the fluorescent pattern of the biochip). The second portion of a first step product was used upon additional purification in sequencing reaction, followed by analysis on an automatic sequencer, correcting a chromatogram and obtaining the sequence of NS5B region fragment, constructing a multiple alignment and a phylogenetic tree on whose basis a genotype and a subtype were determined.

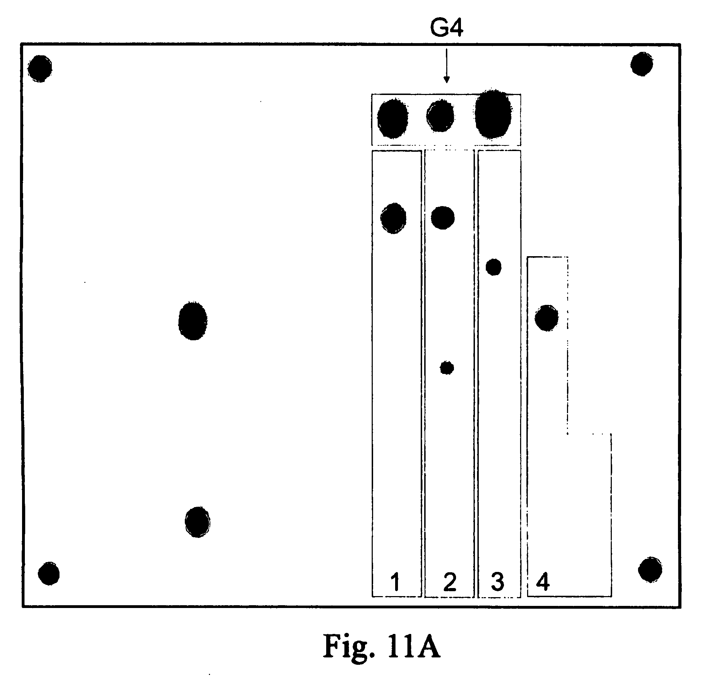

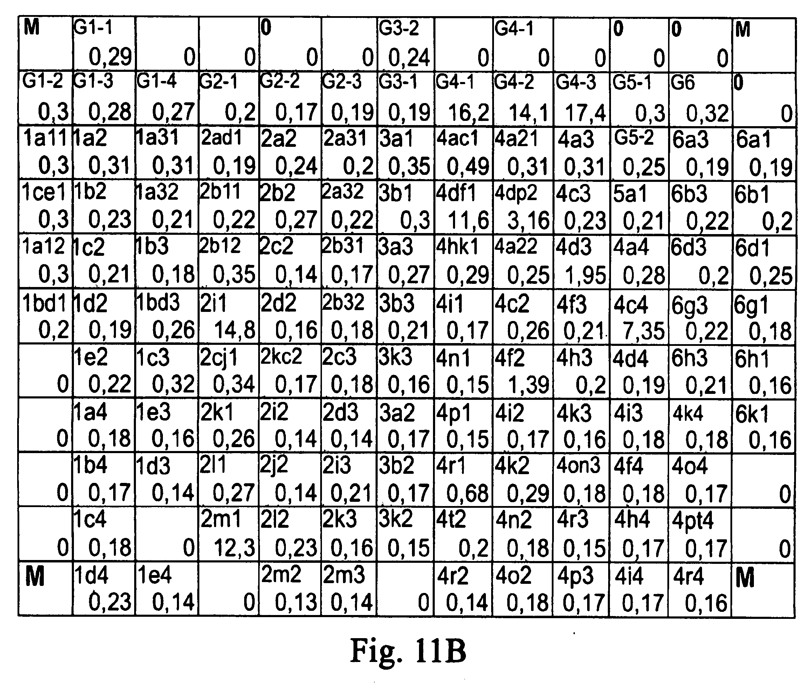

Fig. 11A shows a biochip hybridization pattern. Fig. 11B demonstrates the distribution of the normalized fluorescence signals of biochip elements.

In accordance with the algorithm of results interpretation, as proposed, analysis begins with the computation of a mean signal (I_{ref}) in empty elements. In this case, the value of I_{ref} is 0.35. According to this value and a 1.5 threshold value, the signals are filtered in genotype- and subtype-specific elements. As a result, the signals in G4 group elements containing probes specific for a genotype 4 exceed the I_{ref} 1.5 times or more. The signals in the rest of elements containing genotype-specific oligonucleotides were close to background ones. The maximum signal in the G4 group belongs to a G4-1 element (17.4). It follows that the RNA sequence of a specimen as assayed is related to the genotype 4.

Assaying in the groups of subtype-specific probes of genotype 4 reveals the following points: in group I, the maximum signal is featured by a 4df1 element, (11.6) in group 2 - 4dp2 element (3.16), in group 3 - 4d3 element (1.95), in group 4 - 4d4 (7.35). In all groups, the maximum signal is characteristic of probe-containing elements specific for a subtype 4d. Consequently a specimen as assayed is related to the subtype 4d.

A sequencing method with subsequent phylogenetic analysis showed that the sequence being assayed falls within a cluster of subtype 4d sequences.

Thus, it has been established that an HCV RNA specimen as assayed has a genotype 4 and a subtype 4d, which fully coincides with sequencing results.

### Example 13. Analysis of NS5B region of HCV sample belonging to subtype 5a using hybridization on biochip

An HCV viral RNA was isolated from patient's blood specimen using Qiamp Viral RNA mini kit (Qiagen, Germany) under the protocol of manufacturer. The isolated RNA was used in RT - PCR as described (Example 2). The presence of an amplified NS5B 418 b.p. long fragment was tested by electrophoresis in agarose gel whereupon a RT-PCR product was divided into two portions of which one was treated and assayed according to the methods as described in Examples 2-4 (a second PCR step followed by hybridization on a biochip, washing, registration and interpretation of the fluorescent pattern of the biochip). The second portion of a first step product was used upon additional purification in sequencing reaction, followed by analysis on an automatic sequencer, correcting a chromatogram and obtaining the sequence of NS5B region fragment, constructing a multiple alignment and a phylogenetic tree on whose basis a genotype and a subtype were determined.

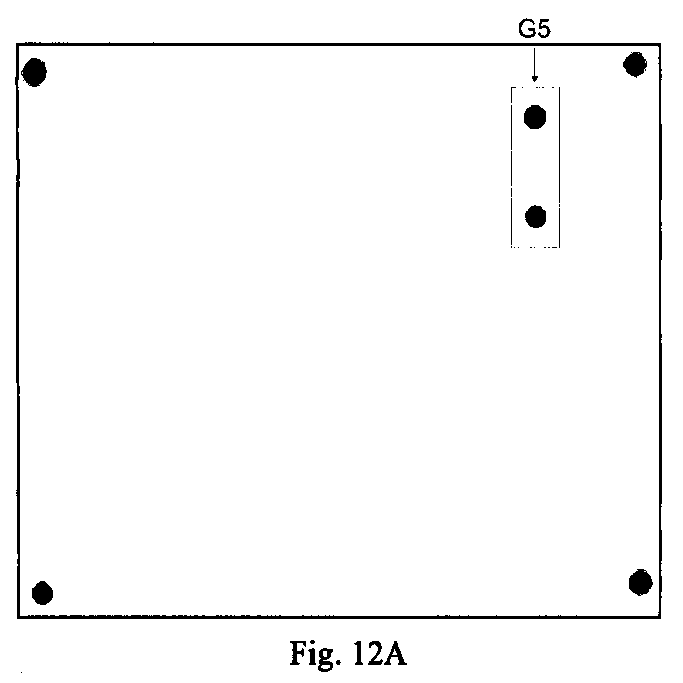

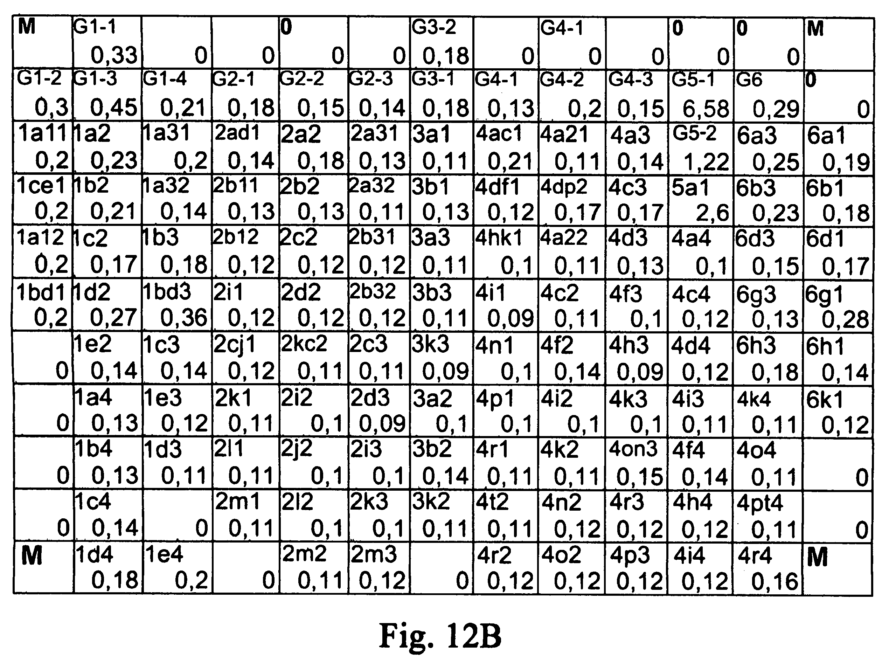

Fig. 12A shows a biochip hybridization pattern. Fig. 12B demonstrates the distribution of the normalized fluorescence signals of biochip elements.

In accordance with the algorithm of results interpretation, as proposed, analysis begins with the computation of a mean signal (I_{ref}) in empty elements. In this case, the value of I_{ref} is 0.28. According to this value and a 1.5 threshold value, the signals are filtered in genotype- and subtype-specific elements, with the result that only the signals in group G5 elements containing probes specific for a genotype 5 exceed the I_{ref} 1.5 times or more. The maximum signal in the G5 group is characteristic of a G5-1 element (6.58). It follows that the RNA sequence of a specimen as assayed is related to the genotype 5. Inasmuch as the latter has only one subtype, 5a, and the signal in a 5a2 element showing a specificity to the given subtype is actual, a conclusion might be drawn that the specimen as assayed has the subtype 5a.

A sequencing method with subsequent phylogenetic analysis showed that the sequence being assayed falls within a cluster of subtype 5a sequences

Thus, it has been established that an HCV RNA specimen as assayed has a genotype 5 and a subtype 5a, which is in full coincidence with sequencing results.

### Example 14. Analysis of NS5B region of HCV sample belonging to genotype 6 using hybridization on biochip

An HCV viral RNA was isolated from patient's blood specimen using Qiamp Viral RNA mini kit (Qiagen, Germany) under the protocol of manufacturer. The isolated RNA was used in RT - PCR as described (Example 2). The presence of an amplified NS5B 418 b.p. long fragment was tested by electrophoresis in agarose gel whereupon a RT-PCR product was divided into two portions of which one was treated and assayed according to the methods as described in Examples 2-4 (a second PCR step followed by hybridization on a biochip, washing, registration and interpretation of the fluorescent pattern of the biochip). The second portion of a first step product was used upon additional purification in sequencing reaction, followed by analysis on an automatic sequencer, correcting a chromatogram and obtaining the sequence of NS5B region fragment, constructing a multiple alignment and a phylogenetic tree on whose basis a genotype and a subtype were determined.

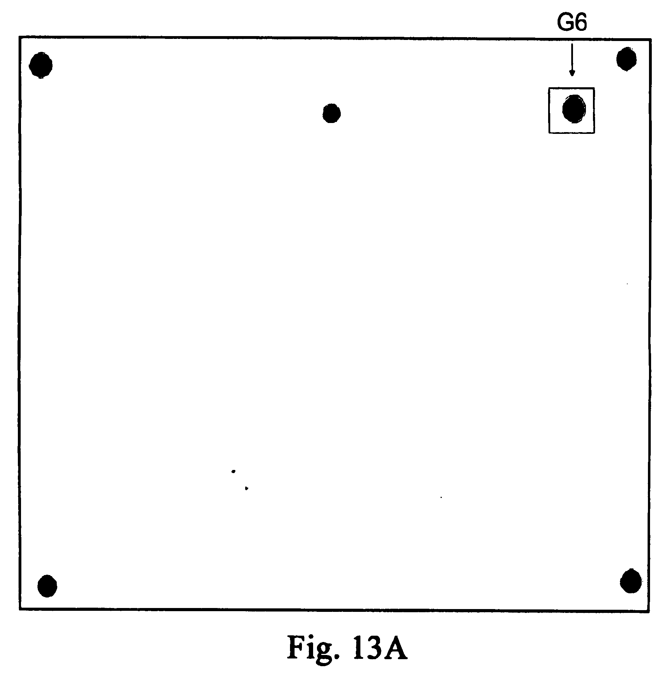

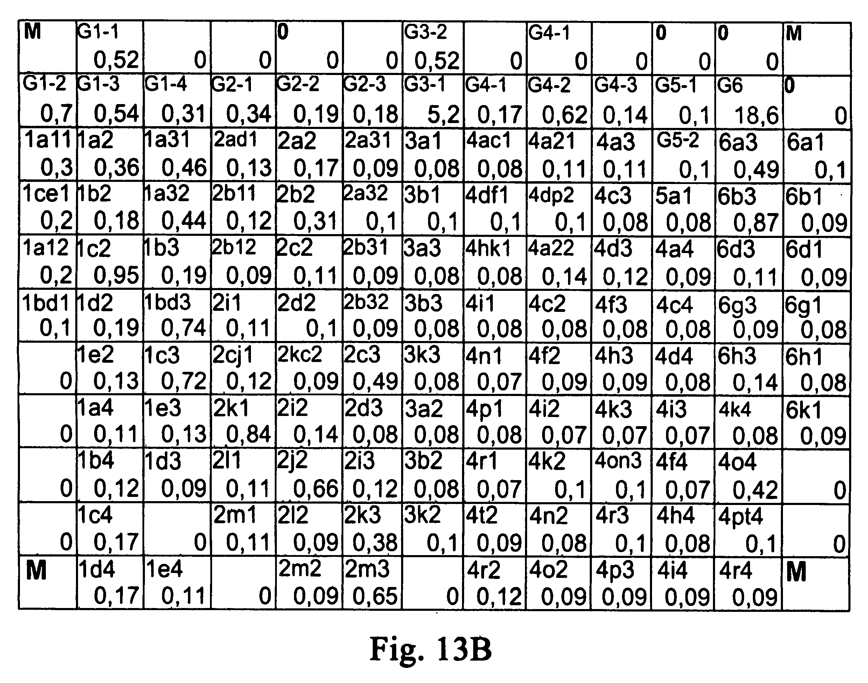

Fig. 13A shows a biochip hybridization pattern. Fig. 13B demonstrates the distribution of the normalized fluorescence signals of biochip elements.

In accordance with the algorithm of results interpretation, as proposed, analysis begins with the computation of a mean signal (I_{ref}) in empty elements. In this case, the value of I_{ref} is 0.72. According to this value and a 1.5 threshold value, signals are filtered in genotype - and subtype-specific elements. As a result, the signals in group G6 element (18.6) comprising a probe showing a specificity to a genotype 6 exceed the I_{ref} 1.5 times or more. The signal in a G3-1 element (5.2) is also to be considered valid. The G6 element exceeds the signal in the G3-1 element more than 1.5 times, which means the identity of analyzed HCV specimen to the genotype 6.

Out of two groups containing probes specific for genotype 6 subtypes, the signal in none of the elements reaches a threshold value relative to I_{ref}. It follows that in the given specimen an HCV subtype is undetermined and is classified as 6x.

A sequencing method with subsequent phylogenetic assay goes to show that the sequence, as assayed, falls within none on the clusters of genotype 6 subtypes and forms a separate branch of a phylogenetic tree, or - to be more exact - it is unclassified. It is hence only logical to see that the results obtained in both methods confirm the impossibility to clearly define the subtype of the given specimen.

Thus, the invention as submitted permits identifying the genotype and subtype of Hepatitis C virus, on the basis of the analysis of an NS5B region using a biological microchip. A method permits identifying all HCV 6 genotypes and 36 subtypes, with the most virulent and drug resistant forms included. The method of the present invention advantageously differs from the existing analogs in high specificity as to the identification of genotype 1 subtypes, more exactly, a 1b subtype, and also in simplicity of execution and low cost. Data obtained through the use of a method of hybridization on biochips of the invention, as being claimed and as set forth in the application, can be used for estimation and prognosis of disease severity (acute/chronic cirrhosis, a likelihood of development of liver cancer), determining a therapeutic dosage of medicaments and duration of a course of therapy as well as for epidemiologic genotyping.

## Claims

1. A method of identifying a genotype and a subtype of hepatitis C virus, on the basis of the analysis of an HCV genome NS5B region, comprising:
(a) - reverse transcription combined with PCR (RT-PCR) using a virus RNA, as a template and a first pair of primers showing a specificity to an NS5B region fragment;
(b) - asymmetric amplification of the NS5B region fragment using as template a RT-PCR product obtained in (a), a second pair of specific primers and a mixture of four deoxynucleoside triphosphates wherein one of the four deoxynucleoside triphosphates is fluorescent labeled, as a substrate, to provide substantially a single-stranded fluorescent labeled fragment;
(c) - providing a biochip for the identification of the HCV genotype and subtype that represents a support comprising a set of discrete elements, with a unique oligonucleotide probe immobilized in each of them, having a sequence complementary to the sequence of a single-stranded fragment obtained in step (b) and selected from the group comprising: a) the NS5B region fragment sequences specific for each of the HCV genotypes (genotype-specific); and b) NS5B region fragment sequences specific for each of the HCV subtypes (subtype-specific);
(d) - hybridization of the amplified labeled product provided in step (b) on said biochip with the formation of duplexes with immobilized probes in conditions providing for a single-nucleotide resolution between the perfect and imperfect duplexes;
(e) - registration and interpretation of hybridization results.

2. The method of claim 1, **characterized in that** in step (a) a first pair of specific primers is used whose sequences are set forth in SEQ ID NO: 121 and 122.

3. The method of claim 1, **characterized in that** in step (b) a second pair of specific primers is used whose sequences are set forth in SEQ ID NO: 121 and 123.

4. The method of claim 1, **characterized in that** in step (b) one of the primers of the second pair is used in an at least tenfold molar excess relative to the second primer.

5. The method of claim 1, **characterized in that** in step (b), the fluorescent labeled deoxynucleoside triphosphate used corresponds to the fluorescent labeled deoxyuridine triphosphate.

6. The method of claim 1, **characterized in that** the biochip is a biochip based on hydrogel elements that is obtained by a procedure of chemically or photoinduced copolymerization.

7. The method of claim 1, **characterized in that** the biochip comprises a set of immobilized oligonucleotides whose sequences are defined in SEQ ID NO: 1-120.

8. The method of claim 1, **characterized in that** registration of the results in step (e) is performed through the use of a portable analyzer of fluorescence and software, which permits using the software-based processing of signal intensities with the subsequent interpretation of results.

9. The method of claim 1, wherein the interpretation of registered results in step (e) is performed in two steps:
1) assaying the signals in biochip elements comprising oligonucleotide probes specific for HCV genotypes thereby to identify the genotype of a specimen as assayed; 2) in the event of identification of a genotype, assayed are only biochip elements containing oligonucleotide probes specific for the subtypes of an identifiable genotype, regardless of presence of the signals in the elements containing the probes specific for the subtypes of other genotypes.

10. The method of claim 1 further comprising evaluating and predicting severity of a disease, , determining a therapeutic dosage of medicaments and a course of therapy and/or epidemiological genotyping on the basis of interpretation of hybridization results.

11. A biochip for identifying an HCV genotype and subtype, on the basis of the analysis of NS5B region comprising a support containing a set of discrete elements, with a unique oligonucleotide probe immobilized in each of them and and whereby the probe sequences are defined in SEQ ID NO: 1-120.

12. The biochip of claim 12, **characterized in that** it is a biochip based on hydrogel elements that is obtained by a procedure of chemically or photoinduced copolymerization.

13. A set of oligonucleotide probes for producing a biochip for the identification of an HCV genotype and subtype, on the basis of the analysis of an NS5B region whose sequences are defined in SEQ ID NO: 1-120.

## Patentansprüche

1. Verfahren zum Identifizieren eines Genotyps und eines Subtyps des Hepatitis C-Virus auf Basis der Analyse einer HCV-Genom-NS5B-Region, umfassend:
(a) - Reverse-Transkription, kombiniert mit PCR (RT-PCR), unter Verwendung einer Virus-RNA als Vorlage und eines ersten Paares an Primern, die eine Spezifizität gegenüber einem NS5B-Regionfragment zeigen;
(b) - asymmetrische Amplifizierung des NS5B-Regionfragments unter Verwendung eines in (a) erhaltenen RT-PCR-Produkts als Vorlage, eines zweiten Paares an spezifischen Primern und eines Gemischs aus vier Desoxynukleosidtriphosphaten, wobei eines der vier Desoxynukleosidtriphosphaten fluoreszenzmarkiert ist, als Substrat, um ein im Wesentlichen einsträngiges fluoreszenzmarkiertes Fragment bereitzustellen;
(c) - Bereitstellen eines Biochips für die Identifizierung des HCV-Genotyps und - Subtyps, der einen Träger darstellt, der einen Satz eigenständiger Elemente umfasst, wobei eine einzigartige Oligonukleotidsonde auf jedem dieser immobilisiert ist, mit einer Sequenz komplementär zur Sequenz eines in Schritt (b) erhaltenen einzelsträngigen Fragments und ausgewählt aus der Gruppe umfassend: a) die NS5B-Regionfragment-Sequenzen, die für jeden der HCV-Genotypen spezifisch sind (genotypspezifisch); und b) NS5B-Regionfragment-Sequenzen, die für jeden der HCV-Subtypen spezifisch sind (subtypspezifisch);
(d) - Hybridisieren des amplifizierten markierten Produkts, das in Schritt (b) bereitgestellt wurde, auf dem Biochip unter Bildung von Duplexen mit immobilisierten Sonden unter Bedingungen, die eine Einzelnukleotidauflösung zwischen den perfekten und nicht-perfekten Duplexen vorsieht;
(e) - Registrieren und Interpretieren von Hybridisierungsergebnissen.

2. Verfahren nach Anspruch 1, **dadurch gekennzeichnet, dass** in Schritt (a) ein erstes Paar an spezifischen Primern verwendet wird, deren Sequenzen in SEQ ID Nr. 121 und 122 angeführt sind.

3. Verfahren nach Anspruch 1, **dadurch gekennzeichnet, dass** in Schritt (b) ein zweites Paar an spezifischen Primern verwendet wird, deren Sequenzen in SEQ ID Nr. 121 und 123 angeführt sind.

4. Verfahren nach Anspruch 1, **dadurch gekennzeichnet, dass** in Schritt (b) einer der Primer des zweiten Paares in einem zumindest zehnfachen molaren Überschuss in Bezug auf den zweiten Primer verwendet wird.

5. Verfahren nach Anspruch 1, **dadurch gekennzeichnet, dass** in Schritt (b) das verwendete fluoreszenzmarkierte Desoxynukleosidtriphosphat dem fluoreszenzmarkierten Desoxyuridintriphosphat entspricht.

6. Verfahren nach Anspruch 1, **dadurch gekennzeichnet, dass** der Biochip ein Biochip auf Hydrogelelementbasis ist, der durch ein Verfahren der chemischen oder lichtinduzierten Copolymerisation erhalten wird.

7. Verfahren nach Anspruch 1, **dadurch gekennzeichnet, dass** der Biochip einen Satz immobilisierter Oligonukleotide umfasst, deren Sequenzen in SEQ ID Nr. 1-120 definiert sind.

8. Verfahren nach Anspruch 1, **dadurch gekennzeichnet, dass** das Registrieren der Ergebnissen in Schritt (e) mithilfe eines tragbaren Fluoreszenzanalysators und Software erfolgt, die die Verwendung der softwarebasierten Verarbeitung von Signalintensitäten mit der darauffolgenden Interpretation der Ergebnisse ermöglicht.

9. Verfahren nach Anspruch 1, wobei die Interpretation der registrierten Ergebnisse in Schritt (e) in zwei Schritten erfolgt:
1) Untersuchen der Signale in Biochipelementen, die die für HCV-Genotypen spezifischen Oligonukleotidsonden umfassen, um dadurch den Genotyp eines untersuchten Prüflings zu identifizieren; 2) im Fall der Identifizierung eines Genotyps Untersuchen von ausschließlich Biochipelementen, die für die Subtypen eines identifizierbaren Genotyps spezifisch sind, unabhängig vom Vorhandensein der Signale in den Elementen, die die für die Subtypen anderer Genotypen spezifischen Sonden enthalten.

10. Verfahren nach Anspruch 1, ferner umfassend das Evaluieren und Prognostizieren des Schweregrads einer Krankheit, das Bestimmen einer therapeutischen Dosis von Medikamenten und den Verlauf einer Therapie und/oder epidemiologisches Genotypisieren auf Basis der Interpretation von Hybridisierungsergebnissen.

11. Biochip zum Identifizieren eines HCV-Genotyps und -Subtyps auf Basis der Analyse einer NS5B-Region, umfassend einen Träger, der einen Satz an eigenständigen Elementen enthält, wobei eine einzigartige Oligonukleotidsonde auf jedem dieser immobilisiert ist, und wobei die Sondensequenzen in SEQ ID Nr. 1-120 definiert sind.

12. Biochip nach Anspruch 12, **dadurch gekennzeichnet, dass** er ein Biochip auf Hydrogelelementbasis ist, der durch ein Verfahren der chemischen oder lichtinduzierten Copolymerisation erhalten wird.

13. Satz Oligonukleotidsonden zum Produzieren eines Biochips für die Identifizierung eines HCV-Genotyps und -Subtyps auf Basis der Analyse einer NS5B-Region, deren Sequenzen in SEQ ID Nr. 1-120 definiert sind.

## Revendications

1. Procédé pour identifier un génotype et un sous-type du virus de l'hépatite C en se basant sur l'analyse d'une région NS5B du génome du VHC, consistant à :
(a) - réaliser une transcription inverse combinée à une PCR (RT-PCR) en utilisant un virus à ARN en tant que matrice et une première paire d'amorces montrant une spécificité pour un fragment de la région NS5B ;
(b) - réaliser une amplification asymétrique du fragment de la région NS5B en utilisant en tant que matrice un produit de RT-PCR obtenu en (a), une deuxième paire d'amorces spécifiques et un mélange de quatre désoxynucléotides triphosphates, dans lequel un des quatre désoxynucléotides triphosphates est marqué par fluorescence, en tant que substrat, afin d'obtenir un fragment marqué par fluorescence essentiellement simple brin ;
(c) - mettre à disposition une biopuce pour l'identification du génotype et du sous-type du VHC, qui représente un support comprenant un ensemble d'éléments discrets avec une sonde oligonucléotidique unique immobilisée sur chacun d'entre eux, ayant une séquence complémentaire à la séquence d'un fragment simple brin obtenu à l'étape (b) et sélectionnée dans le groupe comprenant : a) les séquences du fragment de la région NS5B spécifiques pour chacun des génotypes du VHC (spécifiques au génotype) ; et b) les séquences du fragment de la région NS5B spécifiques pour chacun des sous-types du VHC (spécifiques au sous-type) ;
(d) - réaliser une hybridation du produit marqué amplifié obtenu à l'étape (b) sur ladite biopuce, des duplex étant formés avec les sondes immobilisées dans des conditions permettant une résolution de nucléotides individuels entre les duplex parfaits et imparfaits ;
(e) - réaliser un enregistrement et une interprétation des résultats de l'hybridation.

2. Procédé selon la revendication 1, **caractérisé en ce que** à l'étape (a), une première paire d'amorces spécifiques est utilisée dont les séquences sont décrites dans SEQ ID NO: 121 et 122.

3. Procédé selon la revendication 1, **caractérisé en ce que** à l'étape (b), une deuxième paire d'amorces spécifiques est utilisée dont les séquences sont décrites dans SEQ ID NO: 121 et 123.

4. Procédé selon la revendication 1, **caractérisé en ce que** à l'étape (b), une des amorces de la deuxième paire est utilisée en un excès molaire d'au moins dix fois par rapport à la deuxième amorce.

5. Procédé selon la revendication 1, **caractérisé en ce que** à l'étape (b), le désoxynucléotide triphosphate marqué par fluorescence utilisé correspond au désoxyuridine triphosphate marqué par fluorescence.

6. Procédé selon la revendication 1, **caractérisé en ce que** la biopuce est une biopuce basée sur des éléments d'hydrogel qui est obtenue par une procédure de copolymérisation chimique ou photo-induite.

7. Procédé selon la revendication 1, **caractérisé en ce que** la biopuce comprend un ensemble d'oligonucléotides immobilisés dont les séquences sont définies dans SEQ ID NO: 1-120.

8. Procédé selon la revendication 1, **caractérisé en ce que** l'enregistrement des résultats à l'étape (e) est réalisé en utilisant un analyseur de fluorescence portable et un logiciel, ce qui permet d'utiliser le traitement des intensités de signal par le logiciel avec l'interprétation ultérieure des résultats.

9. Procédé selon la revendication 1, dans lequel l'interprétation des résultats enregistrés à l'étape (e) est réalisée en deux étapes consistant à :
1) analyser les signaux des éléments de la biopuce comprenant les sondes oligonucléotidiques spécifiques aux génotypes du VHC pour ainsi identifier le génotype d'un échantillon analysé ;
2) en cas d'identification d'un génotype, les éléments de la biopuce analysés sont uniquement ceux contenant les sondes oligonucléotidiques spécifiques aux sous-types d'un génotype identifiable, sans tenir compte de la présence des signaux des éléments contenant les sondes spécifiques aux sous-types des autres génotypes.

10. Procédé selon la revendication 1, qui consiste en outre à évaluer et à prédire la sévérité d'une maladie, à déterminer une dose thérapeutique de médicaments et un schéma de traitement et/ou à établir un génotype épidémiologique en se basant sur l'interprétation des résultats de l'hybridation.

11. Biopuce pour identifier un génotype et un sous-type du VHC en se basant sur l'analyse d'une région NS5B, comprenant un support contenant un ensemble d'éléments discrets avec une sonde oligonucléotidique unique immobilisée sur chacun d'entre eux, et moyennant quoi les séquences des sondes sont définies dans SEQ ID NO: 1-120.

12. Biopuce selon la revendication 12, caractérisée comme étant une biopuce basée sur des éléments d'hydrogel qui est obtenue par une procédure de copolymérisation chimique ou photo-induite.

13. Ensemble de sondes oligonucléotidiques pour produire une biopuce destinée à l'identification d'un génotype et d'un sous-type du VHC, en se basant sur l'analyse d'une région NS5B dont les séquences sont définies dans SEQ ID NO: 1-120.
